# EUROPEAN PATENT APPLICATION

(11) **EP 4 455 263 A1**
(43) Date of publication of application: **30.10.2024**
(21) Application number: 22911339.4
(22) Date of filing: 22.12.2022
(51) Int. Cl.: C12N 1/00, C12N 5/07, C12N 7/02, C12P 21/02

(54) **COMPOSITION FOR CULTURING ANIMAL CELLS**

(30) Priority: 24.12.2021 JP 2021211122
(71) Applicant: AJINOMOTO CO., INC., Chuo-ku Tokyo 104-8315 (JP)
(72) Inventor: YAMAIDE, Shinya, Kawasaki-shi, Kanagawa 210-8681 (JP); HARADA, Masashi, Kawasaki-shi, Kanagawa 210-8681 (JP); TASHIRO, Akari, Kawasaki-shi, Kanagawa 210-8681 (JP); TSUJI, Chihiro, Kawasaki-shi, Kanagawa 210-8681 (JP); HIGUCHI, Takuya, Kawasaki-shi, Kanagawa 210-8681 (JP); OKUTANI, Satoshi, Kawasaki-shi, Kanagawa 210-8681 (JP); OGATA, Fumi, Kawasaki-shi, Kanagawa 210-8681 (JP); FUROMITSU, Shumpei, Kawasaki-shi, Kanagawa 210-8681 (JP); SUGIYAMA, Masakazu, Kawasaki-shi, Kanagawa 210-8681 (JP)
(74) Representative: Strehl Schübel-Hopf & Partner
(86) International application number: PCT/JP2022/047331
(87) International publication number: WO 2023/120637

(57) **Abstract**

A composition for animal cell culture and techniques relating thereto are provided. A composition for animal cell culture, which contains an amino acid source and α-ketoglutaric acid, and has the following properties (A) and/or (B): (A) the α-ketoglutaric acid is a salt of α-ketoglutaric acid; and (B) the amino acid source is free L-cysteine anhydrate is provided.

## Description

### Technical Field

The present invention relates to a composition for animal cell culture and techniques relating thereto.

### Background Art

For culturing animal cells, media containing amino acids such as cysteine (Cys) are widely used. Cys is spontaneously oxidized in media and converted into cystine ((Cys)2). Since (Cys)2 has low solubility, it tends to precipitate in liquid media. Therefore, Cys is a factor in the storage instability of liquid media.

Cys can condense with α-keto acids such as pyruvic acid (Pyr) and α-ketoglutaric acid to form thiazolidine derivatives (Non-patent document 1). It has been reported that use of α-keto acids stabilizes the media and that thiazolidine derivatives are effective in culturing animal cells (Non-patent document 1).

### Prior Art Reference

### Non-patent document

Non-patent document 1: Kuschelewski J et al., Antioxidant effect of thiazolidine molecules in cell culture media improves stability and performance. Biotechnol. Prog. 2017 May;33(3):759-770

### Summary of the Invention

### Object to be achieved by the invention

An object of the present invention is to provide a composition for animal cell culture and techniques relating thereto.

### Means for achieving the object

The inventors of the present invention found that problems such as solidification and decrease in cysteine content occur in mixed powders of an amino acid source such as cysteine in a specific form and α-ketoglutaric acid in a specific form, and that these problems can be solved by adjusting the forms of the amino acid source and α-ketoglutaric acid, and thus accomplished the present invention.

The present invention can be embodied, for example, as follows.
[1] A composition for animal cell culture, which
   comprises an amino acid source and α-ketoglutaric acid, and
   wherein the composition satisfies the following conditions (A) and/or (B);
      (A) the α-ketoglutaric acid is a salt of α-ketoglutaric acid; and/or
      (B) the amino acid source is free L-cysteine anhydrate.
[2] The composition mentioned above, wherein the composition satisfies at least the condition (A).
[3] The composition mentioned above, wherein the composition is a powder.
[4] The composition mentioned above, which is a culture medium or culture medium additive.
[5] The composition mentioned above, wherein the culture medium is a basal medium, a feed medium, or a perfusion medium.
[6] The composition mentioned above, wherein the salt of α-ketoglutaric acid is an alkali metal salt or alkaline earth metal salt of α-ketoglutaric acid.
[7] The composition mentioned above, wherein the salt of α-ketoglutaric acid is a sodium salt or potassium salt of α-ketoglutaric acid.
[8] The composition mentioned above, wherein the salt of α-ketoglutaric acid is monosodium α-ketoglutarate or disodium α-ketoglutarate.
[9] The composition mentioned above, wherein the amino acid source is cysteine, glycine, asparagine, glutamic acid, lysine, phenylalanine, methionine, ornithine, tyrosine, or a glycine-containing dipeptide.
[10] The composition mentioned above, wherein the composition satisfies one or more conditions selected from the group consisting of the following conditions (1) to (10):
   (1) the cysteine is free L-cysteine or L-cysteine hydrochloride;
   (2) the glycine is free glycine or free glycyl-L-tyrosine;
   (3) the asparagine is free L-asparagine;
   (4) the glutamic acid is monosodium L-glutamate;
   (5) the lysine is L-lysine hydrochloride;
   (6) the phenylalanine is free L-phenylalanine;
   (7) the methionine is free L-methionine;
   (8) the ornithine is L-ornithine hydrochloride;
   (9) the tyrosine is L-tyrosine disodium salt; and
   (10) the glycine-containing dipeptide is free glycyl-L-tyrosine.
[11] The composition mentioned above, wherein the composition satisfies one or more conditions selected from the group consisting of the following conditions (1a) to (10a):
   (1a) the cysteine is free L-cysteine anhydrate or L-cysteine hydrochloride monohydrate;
   (2a) the glycine is free glycine or free glycyl-L-tyrosine;
   (3a) the asparagine is free L-asparagine monohydrate;
   (4a) the glutamic acid is monosodium L-glutamate monohydrate;
   (5a) the lysine is L-lysine hydrochloride;
   (6a) the phenylalanine is free L-phenylalanine;
   (7a) the methionine is free L-methionine;
   (8a) the ornithine is L-ornithine hydrochloride;
   (9a) the tyrosine is L-tyrosine disodium salt dihydrate; and
   (10a) the glycine-containing dipeptide is free glycyl-L-tyrosine dihydrate.
[12] The composition mentioned above, wherein the amino acid source is cysteine.
[13] The composition mentioned above, wherein the cysteine is free L-cysteine anhydrate or L-cysteine hydrochloride monohydrate.
[14] The composition mentioned above, wherein the cysteine is free L-cysteine anhydrate.
[15] The composition mentioned above, wherein content of the α-ketoglutaric acid is 1 to 20 times content of the amino acid source in terms of molar ratio.
[16] A method for producing a target substance, which comprises
   culturing animal cells having an ability to produce the target substance using the composition mentioned above; and
   collecting the target substance.
[17] The method mentioned above, wherein the target substance is a protein or virus.
[18] A method for culturing animal cells, which comprises
   culturing animal cells by using the composition mentioned above.
[19] Use of a salt of α-ketoglutaric acid for preventing solidification of a composition containing an amino acid source.
[20] The use mentioned above, wherein the amino acid source is cysteine, glycine, asparagine, glutamic acid, lysine, phenylalanine, methionine, ornithine, tyrosine, or a glycine-containing dipeptide.
[21] The use mentioned above, wherein one or more conditions selected from the following conditions (1) to (10) is/are satisfied:
   (1) the cysteine is free L-cysteine or L-cysteine hydrochloride;
   (2) the glycine is free glycine or free glycyl-L-tyrosine;
   (3) the asparagine is free L-asparagine;
   (4) the glutamic acid is monosodium L-glutamate;
   (5) the lysine is L-lysine hydrochloride;
   (6) the phenylalanine is free L-phenylalanine;
   (7) the methionine is free L-methionine;
   (8) the ornithine is L-ornithine hydrochloride;
   (9) the tyrosine is L-tyrosine disodium salt; and
   (10) the glycine-containing dipeptide is free glycyl-L-tyrosine.
[22] The use mentioned above, wherein one or more conditions selected from the following conditions (1a) to (10a) is/are satisfied:
   (1a) the cysteine is free L-cysteine anhydrate or L-cysteine hydrochloride monohydrate;
   (2a) the glycine is free glycine or free glycyl-L-tyrosine;
   (3a) the asparagine is free L-asparagine monohydrate;
   (4a) the glutamic acid is monosodium L-glutamate monohydrate;
   (5a) the lysine is L-lysine hydrochloride;
   (6a) the phenylalanine is free L-phenylalanine;
   (7a) the methionine is free L-methionine;
   (8a) the ornithine is L-ornithine hydrochloride;
   (9a) the tyrosine is L-tyrosine disodium salt dihydrate; and
   (10a) the glycine-containing dipeptide is free glycyl-L-tyrosine dihydrate.
[23] The use mentioned above, wherein the amino acid source is cysteine.
[24] Use of a salt of α-ketoglutaric acid for preventing decrease in cysteine content in a composition containing cysteine.
[25] The use mentioned above, wherein the salt of α-ketoglutaric acid is an alkali metal salt or alkaline earth metal salt of α-ketoglutaric acid.
[26] The use mentioned above, wherein the salt of α-ketoglutaric acid is a sodium salt or potassium salt of α-ketoglutaric acid.
[27] The use mentioned above, wherein the salt of α-ketoglutaric acid is monosodium α-ketoglutarate or disodium α-ketoglutarate.
[28] The use mentioned above, wherein the cysteine is free L-cysteine anhydrate or L-cysteine hydrochloride monohydrate.
[29] The use mentioned above, wherein the cysteine is free L-cysteine anhydrate.
[30] The use mentioned above, wherein the salt of α-ketoglutaric acid is used so that content of the salt of α-ketoglutaric acid in the composition is 1 to 20 times content of the amino acid source or the cysteine in the composition in terms of molar ratio.
[31] A method for producing a composition for animal cell culture, wherein
   the composition is the composition according to any one of [1] to [15], and
   the method comprises mixing the amino acid source and the α-ketoglutaric acid.

### Brief Description of the Drawings

[Fig. 1] Drawing (photographs) showing degrees of solidification of mixed powders of L-cysteine and α-ketoglutaric acid stored at 25°C.
[Fig. 2] Drawing (photographs) showing degrees of solidification of mixed powders of L-cysteine and α-ketoglutaric acid stored at 40°C.
[Fig. 3] Drawing (photographs) showing degrees of solidification of mixed powders of various amino acid sources and α-ketoglutaric acid immediately after preparation.
[Fig. 4] Drawing (photographs) showing degrees of solidification of mixed powders of various amino acid sources and α-ketoglutaric acid stored at 4°C.
[Fig. 5] Drawing (photographs) showing degrees of solidification of mixed powders of various amino acid sources and α-ketoglutaric acid stored at 25°C.
[Fig. 6] Drawing (photographs) showing degrees of solidification of mixed powders of various amino acid sources and α-ketoglutaric acid stored at 40°C.
[Fig. 7] Drawing showing changes in L-cysteine content in a mixed powder of L-cysteine hydrochloride monohydrate and free α-ketoglutaric acid stored at 25°C or 40°C.
[Fig. 8] Drawing showing changes in L-cysteine content in a mixed powder of free L-cysteine anhydrate and free α-ketoglutaric acid stored at 25°C or 40°C.
[Fig. 9] Drawing showing changes in L-cysteine content in a mixed powder of L-cysteine hydrochloride monohydrate and monosodium α-ketoglutarate stored at 25°C or 40°C.
[Fig. 10] Drawing showing changes in L-cysteine content in a mixed powder of free L-cysteine anhydrate and monosodium α-ketoglutarate stored at 25°C or 40°C.

### Modes for Carrying out the Invention

### <1> Composition of the present invention

The composition of the present invention is a composition comprising an amino acid source and α-ketoglutaric acid.

The amino acid source and α-ketoglutaric acid are also collectively referred to as "active ingredients".

The composition of the present invention can be used for, for example, culture of animal cells. That is, the composition of the present invention may be a composition for animal cell culture. Specifically, the composition of the present invention can be used for, for example, culture of animal cells in such a manner as described in the section of Method of the present invention described later.

The composition of the present invention may be, for example, a culture medium. Examples of the culture medium include a basal medium, a feed medium, and a perfusion medium. The term "basal medium" may mean a medium used at the start of culture. The basal medium is also referred to as "starting medium". The term "feed medium" may refer to a medium supplied to the culture system after the start of culture in fed-batch culture. The term "perfusion medium" may mean a medium supplied to the culture system after the start of the culture in continuous culture (which is not limited to perfusion culture).

The composition of the present invention may be, for example, a culture medium additive. The term "culture medium additive" may mean a composition that is used by adding it to a culture medium. Examples of the medium to which the culture medium additive is added include a basal medium, a feed medium, and a perfusion medium.

The composition of the present invention satisfies the following conditions (A) and/or (B):
(A) the α-ketoglutaric acid is a salt of α-ketoglutaric acid; and
(B) the amino acid source is free L-cysteine anhydrate.

The composition of the present invention may satisfy only one of the conditions (A) and (B), or may satisfy both the conditions (A) and (B). The composition of the present invention may satisfy, especially, at least the condition (A). The composition of the present invention may satisfy, further especially, both the conditions (A) and (B).

Because the composition of the present invention satisfies the conditions (A) and/or (B), stability of the composition of the present invention can be improved, i.e. an effect of improving stability of the composition of the present invention can be obtained. This effect is also referred to as "stability-improving effect". Specifically, stability of the composition of the present invention having the conditions (A) and/or (B) can be improved compared with the case where the composition of the present invention does not satisfy the conditions (A) and/or (B).

Examples of improvement of stability of the composition include prevention of solidification of the composition. When the composition of the present invention contains cysteine (specifically, when cysteine is selected as the amino acid source which is one of the active ingredients), examples of improvement of stability of the composition also include prevention of decrease in cysteine content in the composition. "Prevention of solidification of the composition" means to reduce the degree of solidification of the composition, and includes making the composition not solidify at all. "Prevention of decrease in cysteine content in the composition" means to reduce the degree of decrease in cysteine content in the composition, and includes making the cysteine content in the composition not decrease at all. The effect of preventing solidification of the composition is also referred to as the "solidification-preventing effect". The effect of preventing decrease in cysteine content in the composition is also referred to as "cysteine decrease-preventing effect". That is, because the composition of the present invention satisfies the conditions (A) and/or (B), the solidification-preventing effect and/or the cysteine decrease-preventing effect may be obtained. In other words, because the composition of the present invention satisfies the conditions (A) and/or (B), solidification of the composition may be prevented and/or decrease in cysteine content in the composition may be prevented. Specifically, in the composition of the present invention having the conditions (A) and/or (B), solidification of the composition may be more prevented and/or decrease in cysteine content in the composition may be more prevented compared with the case where the composition of the present invention does not satisfy the conditions (A) and/or (B).

For example, stability of the composition of the present invention having only one of the conditions (A) and (B) may be improved compared with the case where the composition of the present invention had neither the condition (A) nor (B). In particular, solidification of the composition of the present invention having only one of the conditions (A) and (B) may be more prevented compared with the case where the composition of the present invention had neither the condition (A) nor (B).

For example, stability of the composition of the present invention having both the conditions (A) and (B) may be improved compared with the case where the composition of the present invention had neither the condition (A) nor (B). In the composition of the present invention having both the conditions (A) and (B), especially, solidification of the composition may be more prevented and/or decrease in cysteine content in the composition may be more prevented compared with the case where the composition of the present invention had neither the condition (A) nor (B). In the composition of the present invention having both the conditions (A) and (B), further especially, solidification of the composition may be more prevented, and decrease in cysteine content in the composition may be more prevented compared with the case where the composition of the present invention had neither the condition (A) nor (B).

For example, stability of the composition of the present invention having both the conditions (A) and (B) may be improved compared with the case where the composition of the present invention satisfies only one of the conditions (A) and (B). In the composition having both the conditions (A) and (B), especially, solidification of the composition may be more prevented and/or decrease in cysteine content in the composition may be more prevented compared with the case where the composition of the present invention satisfies only one of the conditions (A) and (B). In the composition having both the conditions (A) and (B), further especially, solidification of the composition of the present invention may be more prevented and decrease in cysteine content in the composition may be more prevented compared with the case where the composition of the present invention satisfies only one of the conditions (A) and (B).

Examples of the composition of the present invention that does not satisfy the condition (A) include the composition in which the α-ketoglutaric acid is α-ketoglutaric acid in the free form. Examples of the composition of the present invention that does not satisfy the condition (B) include the composition in which the amino acid source is cysteine other than free L-cysteine, specifically the composition in which the amino acid source is L-cysteine hydrochloride monohydrate or the amino acid source is L-cysteine hydrochloride anhydrate.

The stability-improving effect can be confirmed by comparing the relevant parameters of the compositions of the present invention having and not having the conditions (A) and/or (B) after storage under predetermined conditions.

In other words, the solidification-preventing effect can be confirmed by comparing degrees of solidification of the compositions of the present invention having and not having the conditions (A) and/or (B) after storage under predetermined conditions. The degree of solidification can be confirmed by, for example, visual inspection with the naked eye. The degree of solidification can also be confirmed by, for example, using the particle diameter of the composition (e.g., average particle diameter D50) as an indicator. That is, it may be determined that the smaller the particle diameter of the composition (e.g., average particle diameter D50), the lower the degree of solidification. The term "average particle diameter D50" may mean the particle diameter corresponding to 50% of the % passing mass fraction in a graph of the cumulative particle diameter curve. The average particle diameter D50 can be measured by using, for example, a low-tap sieve shaker.

The cysteine decrease-preventing effect can be confirmed by comparing degrees of decrease in cysteine content in the compositions of the present invention having and not having the conditions (A) and/or (B) after storage under predetermined conditions. The degree of decrease in cysteine content can be confirmed by using the ratio of cysteine contents in the composition of the present invention before and after storage as an indicator. That is, it can be determined that the greater the ratio of the cysteine content in the composition of the present invention after storage to the cysteine content in the composition of the present invention before storage, the lower the degree of decrease in cysteine content. The cysteine content can be measured by, for example, the method described in WO2021/060517A.

Examples of the predetermined conditions for storage mentioned above include conditions under which solidification and/or decrease in cysteine content is observed if the composition of the present invention had neither the condition (A) nor (B). Specifically, examples of the storage under the predetermined conditions include storages at 25°C for 3 days, 25°C for 7 days, 25°C for 14 days, 25°C for 21 days, 40°C for 3 days, 40°C for 7 days, 40°C for 14 days, and 40°C for 21 days under light-shielding conditions. The storage may be performed, for example, in the presence of a moisture-absorbing agent. Examples of the moisture-absorbing agent include silica gel, quicklime (calcium oxide), calcium chloride, and zeolite. Silica gel is a particularly good example of the moisture-absorbing agent. The storage may also be carried out, for example, within a packaging material having a moisture-absorbing function. Examples of the packaging material having a moisture-absorbing function include an aluminum pouch having a moisture-absorbing film. More specific examples of the storage under the predetermined conditions include storages at 25°C for 3 days, 25°C for 7 days, 25°C for 14 days, 25°C for 21 days, 40°C for 3 days, 40°C for 7 days, 40°C for 14 days, and 40°C for 21 days under light-shielding conditions in the presence of a moisture absorbing agent or within a packaging material having a moisture-absorbing function. The stability-improving effect (e.g., solidification-preventing effect and/or cysteine decrease-preventing effect) may be confirmed, for example, under one or more types of conditions selected from the conditions exemplified above.

The term "amino acid source" is a generic term for amino acids and compounds that generate them. The amino acid source is not particularly limited so long as the stability-improving effect can be obtained. The amino acid source may be, for example, one that causes solidification in a mixture thereof with free α-ketoglutaric acid. Whether or not solidification occurs in a mixture of the amino acid source and free α-ketoglutaric acid can be confirmed by checking whether or not solidification occurs in the mixture after storage under predetermined conditions. As for the predetermined conditions for storage, the description for the predetermined conditions for storage for confirming the stability-improving effect mentioned above can be applied.

Examples of the amino acid source include glycine source, alanine source, valine source, leucine source, isoleucine source, cysteine source, methionine source, phenylalanine source, tyrosine source, tryptophan source, histidine source, lysine source, arginine source, serine source, threonine source, aspartic acid source, glutamic acid source, asparagine source, glutamine source, proline source, and ornithine source. That is, examples of the amino acid include glycine, alanine, valine, leucine, isoleucine, cysteine, methionine, phenylalanine, tyrosine, tryptophan, histidine, lysine, arginine, serine, threonine, aspartic acid, glutamic acid, asparagine, glutamine, proline, and ornithine. Examples of the amino acid source include, especially, cysteine source, glycine source, asparagine source, glutamic acid source, lysine source, phenylalanine source, methionine source, ornithine source, and tyrosine source. That is, examples of the amino acids include, especially, cysteine, glycine, asparagine, glutamic acid, lysine, phenylalanine, methionine, ornithine, and tyrosine. Examples of the amino acid source include, further especially, cysteine source, glycine source, asparagine source, glutamic acid source, lysine source, phenylalanine source, and methionine source. That is, examples of the amino acid include, further especially, cysteine, glycine, asparagine, glutamic acid, lysine, phenylalanine, and methionine. Examples of the amino acid source include, still further especially, cysteine source. That is, examples of the amino acid include, still further especially, cysteine. Examples of the compounds that generate an amino acid include compounds that generate an amino acid by hydrolysis. Examples of the compounds that generate an amino acid by hydrolysis include peptides containing an amino acid as a component. Examples of the peptides include dipeptides and tripeptides. Examples of the peptides include, especially, peptides containing glycine as a component, such as dipeptides containing glycine as a component. A peptide containing glycine as a component is also refers to as "glycine-containing peptide". The position of glycine in the glycine-containing peptide is not particularly restricted. The position of glycine in the glycine-containing peptide may be, for example, N-terminus, C-terminus, or other positions. The position of glycine in the glycine-containing peptide may be, especially, N-terminus. Examples of the glycine-containing dipeptide include, especially, dipeptides of glycine and any of the amino acids exemplified above. Examples of the glycine-containing dipeptide include, further especially, glycyltyrosine. Glycyltyrosine is an example of the glycine source and also an example of the tyrosine source. Examples of the amino acid source (which may be, for example, one that causes solidification in a mixture thereof with free α-ketoglutaric acid) include, especially, cysteine, glycine, asparagine, glutamic acid, lysine, phenylalanine, methionine, ornithine, tyrosine, and glycine-containing dipeptides. Examples of the amino acid source (which may be, for example, one that causes solidification in a mixture thereof with free α-ketoglutaric acid) include, further especially, cysteine, glycine, asparagine, glutamic acid, lysine, phenylalanine, methionine, and glycine-containing dipeptides. Further, in order to obtain the cysteine decrease-preventing effect, examples of the amino acid source include, especially, cysteine.

As the amino acid source, a single type of amino acid source or a combination of two or more types of amino acid sources may be used. As the amino acid source, for example, a combination of a cysteine source and one or more types of other amino acid sources may be used. As the amino acid source, specifically, for example, a combination of a cysteine source and one or more types of other amino acid sources selected from glycine source, asparagine source, glutamic acid source, lysine source, phenylalanine source, methionine source, ornithine source, and tyrosine source can be used. As the amino acid source, specifically, for example, a combination of a cysteine source and one or more types of other amino acid sources selected from glycine source, asparagine source, glutamic acid source, lysine source, phenylalanine source, and methionine source may be used.

The amino acid may be, for example, an L-amino acid. That is, for example, alanine, valine, leucine, isoleucine, cysteine, methionine, phenylalanine, tyrosine, tryptophan, histidine, lysine, arginine, serine, threonine, aspartic acid, glutamic acid, asparagine, glutamine, proline, and ornithine may be L-alanine, L-valine, L-leucine, L-isoleucine, L-cysteine, L-methionine, L-phenylalanine, L-tyrosine, L-tryptophan, L-histidine, L-lysine, L-arginine, L-serine, L-threonine, L-aspartic acid, L-glutamic acid, L-asparagine, L-glutamine, L-proline, and L-ornithine, respectively. Further, for example, glycyltyrosine may be glycyl-L-tyrosine.

If the amino acid source can form a salt, the amino acid source may be used as a compound in the free form or salt, or a combination thereof. That is, the term "amino acid source" may mean an amino acid source as a free compound, a salt thereof, or a combination of them, unless otherwise noted. For example, the term "cysteine" may mean free cysteine, a salt thereof, or a combination of them, unless otherwise noted. The term "free compound " means a compound that is not forming a salt. The salt is not particularly limited so long as it is a salt that can be used for animal cell culture. For example, examples of salts for acidic groups such as carboxyl group include ammonium salts, salts with alkali metals such as sodium and potassium, salts with alkaline earth metals such as calcium and magnesium, aluminum salts, zinc salts, salts with organic amines such as triethylamine, ethanolamine, morpholine, pyrrolidine, piperidine, piperazine, and dicyclohexylamine, and salts with basic amino acids such as arginine and lysine. Examples of salts for basic groups such as amino group include salts with inorganic acids such as hydrochloric acid, sulfuric acid, phosphoric acid, nitric acid, and hydrobromic acid, salts with organic carboxylic acids such as acetic acid, citric acid, benzoic acid, maleic acid, fumaric acid, tartaric acid, succinic acid, tannic acid, butyric acid, hibenzic acid, pamoic acid, enanthic acid, decanoic acid, theoclic acid, salicylic acid, lactic acid, oxalic acid, mandelic acid, and malic acid, and salts with organic sulfonic acids such as methanesulfonic acid, benzene sulfonic acid, and p-toluenesulfonic acid. As the salt, one type of salt may be used, or a combination of two or more types of salts may be used. For example, examples of salts of cysteine, lysine, or ornithine include, especially, salts with inorganic acids. Examples of salts of cysteine, lysine, or ornithine include, further especially, hydrochlorides. For example, examples of salt of tyrosine or glutamic acid include, especially, salts with alkali metals. Examples of salts of tyrosine or glutamic acid include, further especially, sodium salts. Examples of sodium salts of tyrosine include disodium salt. Examples of the sodium salts of glutamic acid include monosodium salt. When the amino acid source can form a salt, the amino acid source may be used as an anhydrate, a hydrate, or a combination thereof. That is, the term "amino acid source" (e.g., "amino acid source as a free compound" or "salt of the amino acid source") may also mean anhydrate or hydrate, unless otherwise noted. For example, the term "cysteine" (e.g., "free cysteine" or "salt of cysteine") may include anhydrate and hydrate, unless otherwise noted. For example, examples of hydrate of cysteine, asparagine, or glutamic acid include monohydrates thereof. For example, examples of hydrate of glycyltyrosine include dihydrate. When the composition of the present invention is used (e.g., at the time of animal cell culture), the amino acid source may be in a form corresponding to the way of use of the composition such as in the form of ion.

Examples of the cysteine source (specifically cysteine) include, especially, L-cysteine. Examples of L-cysteine include, especially, free L-cysteine and L-cysteine hydrochloride. Examples of free L-cysteine include, especially, free L-cysteine anhydrate. Examples of L-cysteine hydrochloride include, especially, L-cysteine hydrochloride monohydrate. That is, examples of the cysteine source include, further especially, free L-cysteine anhydrate and L-cysteine hydrochloride monohydrate. Examples of the cysteine source include, further especially, free L-cysteine anhydrate. When the condition (B) is selected, the cysteine source is free L-cysteine anhydrate.

Examples of the glycine source (specifically glycine or dipeptides containing it as a component) include, especially, glycine and glycyl-L-tyrosine. Examples of glycine include, especially, free glycine. Examples of glycyl-L-tyrosine include, especially, free glycyl-L-tyrosine. Examples of free glycyl-L-tyrosine include, especially, free glycyl-L-tyrosine dihydrate.

Examples of the asparagine source (specifically, asparagine) include, especially, L-asparagine. Examples of L-asparagine include, especially, free L-asparagine. Examples of free L-asparagine include, especially, free L-asparagine monohydrate.

Examples of the glutamic acid source (specifically, glutamic acid) include, especially, L-glutamic acid. Examples of L-glutamic acid include, especially, monosodium L-glutamate. Examples of monosodium L-glutamate include, especially, monosodium L-glutamate monohydrate.

Examples of the lysine source (specifically, lysine) include, especially, L-lysine. Examples of L-lysine include, especially, L-lysine hydrochloride.

Examples of the phenylalanine source (specifically, phenylalanine) include, especially, L-phenylalanine. Examples of L-phenylalanine include, especially, free L-phenylalanine.

Examples of the methionine source (specifically methionine) include, especially, L-methionine. Examples of L-methionine include, especially, free L-methionine.

Examples of the ornithine source (specifically ornithine) include, especially, L-ornithine. Examples of L-ornithine include, especially, L-ornithine hydrochloride.

Examples of the tyrosine source (specifically, tyrosine or dipeptides containing it as a component) include, especially, L-tyrosine and glycyl-L-tyrosine. Examples of L-tyrosine include, especially, free L-tyrosine and L-tyrosine disodium salt. Examples of L-tyrosine disodium salt include L-tyrosine disodium salt dihydrate. Examples of glycyl-L-tyrosine include, especially, free glycyl-L-tyrosine. Examples of free glycyl-L-tyrosine include, especially, free glycyl-L-tyrosine dihydrate.

That is, the composition of the present invention may satisfy, for example, one or more conditions selected from the group consisting of the following conditions (1) to (10):
(1) the cysteine is free L-cysteine or L-cysteine hydrochloride;
(2) the glycine is free glycine or free glycyl-L-tyrosine;
(3) the asparagine is free L-asparagine;
(4) the glutamic acid is monosodium L-glutamate;
(5) the lysine is L-lysine hydrochloride;
(6) the phenylalanine is free L-phenylalanine;
(7) the methionine is free L-methionine;
(8) the ornithine is L-ornithine hydrochloride;
(9) the tyrosine is L-tyrosine disodium salt; and
(10) the glycine-containing dipeptide is free glycyl-L-tyrosine.

The composition may also satisfy, for example, one or more conditions selected from the group consisting of the following conditions (1a) to (10a):
(1a) the cysteine is free L-cysteine anhydrate or L-cysteine hydrochloride monohydrate;
(2a) the glycine is free glycine or free glycyl-L-tyrosine;
(3a) the asparagine is free L-asparagine monohydrate;
(4a) the glutamic acid is monosodium L-glutamate monohydrate;
(5a) the lysine is L-lysine hydrochloride;
(6a) the phenylalanine is free L-phenylalanine;
(7a) the methionine is free L-methionine;
(8a) the ornithine is L-ornithine hydrochloride;
(9a) the tyrosine is L-tyrosine disodium salt dihydrate; and
(10a) the glycine-containing dipeptide is free glycyl-L-tyrosine dihydrate.

The conditions (1a) to (10a) mentioned above may be examples of the conditions (1) to (10) mentioned above, respectively.

The amino acid source may be a commercially available product or one obtained by appropriately producing it. The method for producing the amino acid source is not particularly restricted. The amino acid source can be produced by, for example, chemical synthesis, enzymatic reaction, fermentation, extraction, or a combination thereof. Specifically, the amino acid source can be produced by, for example, culturing a microorganism having an ability to produce an amino acid source and collecting the amino acid source from the culture medium or the cells. The amino acid source may or may not be purified to a desired degree. In other words, as the amino acid source, a purified product may be used, or a material containing the amino acid source may be used. For example, a material having an amino acid source content of 1% (w/w) or higher, 5% (w/w) or higher, 10% (w/w) or higher, 30% (w/w) or higher, 50% (w/w) or higher, 70% (w/w) or higher, 90% (w/w) or higher, or 95% (w/w) or higher of the may be used as the amino acid source.

The α-ketoglutaric acid may be used as free compound, a salt, or a combination thereof. That is, the term "α-ketoglutaric acid" may mean free α-ketoglutaric acid, a salt thereof, or a combination thereof, unless otherwise noted. For the salt of α-ketoglutaric acid, the description for salts of the amino acid source for acidic groups can be applied. Examples of the salt of α-ketoglutaric acid include, especially, salts with alkali metals and salts with alkaline earth metals. Examples of the salt of α-ketoglutaric acid include, further especially, salts with alkali metals such as sodium salts and potassium salts. Examples of the salt of α-ketoglutaric acid include, further especially, sodium salts. Examples of the sodium salts of α-ketoglutaric acid include, especially, monosodium salts and disodium salts. Examples of the sodium salts of α-ketoglutaric acid include, especially, monosodium salts. Examples of the potassium salts of α-ketoglutaric acid include monopotassium salts and dipotassium salts. In addition, α-ketoglutaric acid may be used as an anhydrate, a hydrate, or a combination thereof. That is, the term "α-ketoglutaric acid" (e.g., "free α-ketoglutaric acid" and "salt of α-ketoglutaric acid") may encompass anhydrate and hydrate, unless otherwise noted. Examples of the hydrate of α-ketoglutaric acid include monohydrate and dihydrate. Specific examples of α-ketoglutaric acid (especially salt of α-ketoglutaric acid) include monosodium α-ketoglutarate anhydrate and disodium α-ketoglutarate dihydrate. When the condition (A) is selected, α-ketoglutaric acid is a salt of α-ketoglutaric acid. At the time of using the composition of the present invention (e.g., at the time of culturing animal cells), the α-ketoglutaric acid may be in a form corresponding to the way of use of the composition, such as in the form of ion.

The α-ketoglutaric acid may be a commercially available product or one obtained by appropriately producing it. The method for producing α-ketoglutaric acid is not particularly limited. The α-ketoglutaric acid may be produced by, for example, chemical synthesis, enzymatic reaction, fermentation, extraction, or a combination thereof. Specifically, the α-ketoglutaric acid can be produced by, for example, culturing a microorganism having an ability to produce α-ketoglutaric acid and collecting α-ketoglutaric acid from the culture medium or the cells. The α-ketoglutaric acid may or may not be purified to a desired degree. In other words, as the α-ketoglutaric acid, a purified product may be used or a material containing α-ketoglutaric acid may be used. As the α-ketoglutaric acid, for example, a material having an α-ketoglutaric acid content of 1% (w/w) or higher, 5% (w/w) or higher, 10% (w/w) or higher, 30% (w/w) or higher, 50% (w/w) or higher, 70% (w/w) or higher, 90% (w/w) or higher, or 95% (w/w) or higher may be used.

The composition may consist of the active ingredients or may contain an ingredient other than the active ingredients. The ingredient other than the active ingredients is also referred to as "additional ingredient".

The additional ingredient is not particularly restricted so long as it does not impair the purpose of the present invention. The additional ingredient can be appropriately selected according to various conditions, for example, the type of animal cells and the manner in which the composition of the present invention is used. Examples of the additional ingredient include culture medium components.

Examples of the culture medium components include carbon source, amino acid source, vitamin, inorganic component, pH buffer, growth factor, serum, serum albumin, selective agent, and gene expression inducer. Examples of the carbon source include sugars such as glucose. The amino acid source is as described above. The amino acid source as the additional ingredient may or may not be, for example, one that causes solidification in a mixture thereof with free α-ketoglutaric acid. The amino acid source as the additional ingredient may be one that has not been selected as the amino acid source as the active ingredients. For example, if cysteine is selected as the active ingredient amino acid source, one or more types of amino acid sources other than cysteine may be selected as the additional ingredient. Examples of the additional ingredient amino acid source include, especially, tyrosine source. Examples of the vitamin include vitamin A, vitamin B 1, vitamin B2, vitamin B3, vitamin B5, vitamin B6, vitamin B7, vitamin B9, vitamin B 12, vitamin C, vitamin D, vitamin E, vitamin K, and precursors thereof. Examples of the inorganic component include sodium, potassium, calcium, magnesium, phosphorus, and various trace elements (e.g., Co, Cu, F, Fe, Mn, Mo, Ni, Se, Si, Ni, Bi, V, and Zn). Examples of the pH buffer include sodium hydrogencarbonate, phosphates, and HEPES. Examples of the growth factor include insulin, IGF-1, and FGF.

If the additional ingredient can form a salt, the additional ingredient may be used as a free compound, a salt, or a combination thereof. In other words, the term "additional ingredient" may mean the additional ingredient in the form of a free compound, a salt thereof, or a combination thereof, unless otherwise noted. For the salt of additional ingredient, the descriptions for salt of amino acid source and salt of α-ketoglutaric acid can be applied. If the additional ingredient can form a hydrate, the additional ingredient may be used as an anhydrate, a hydrate, or a combination thereof. That is, the term "additional ingredient" (e.g., "additional ingredient in the form of a free compound" or "salt of additional ingredient") may encompass anhydrate and hydrate, unless otherwise noted. For the hydrate of the additional ingredient, the descriptions for hydrate of amino acid source and hydrate of α-ketoglutaric acid can be applied. The additional ingredient may be in a form suitable for the manner in which the composition of the present invention is used, such as in the form of ion.

In one embodiment, because the composition of the present invention satisfies the condition (A), solidification of the composition of the present invention that may result from the presence of an additional ingredient such as amino acid source may be prevented.

As the additional ingredient, one type of ingredient may be used, or a combination of two or more types of ingredients may be used.

The composition of the present invention can be produced by, for example, appropriately mixing the active ingredients and optional additional ingredient. In other words, examples of the method for producing the composition of the present invention include a method for producing the composition of the present invention comprising mixing the active ingredients (i.e., mixing the amino acid source and α-ketoglutaric acid). Each active ingredient may or may not be pre-mixed with an additional ingredient prior to mixing with the other active ingredient. In other words, each of the active ingredients in the expression "mixing the active ingredients" may be one pre-mixed with an additional ingredient. The additional ingredient may also further be mixed after the active ingredients are mixed.

The composition of the present invention may be, for example, appropriately formulated into a preparation. When the composition of the present invention is formulated into a preparation, an additive may be appropriately used. Examples of the additive include excipient, binder, disintegrating agent, lubricant, stabilizer, flavoring agent, diluent, and surfactant. The additive can be appropriately selected depending on various conditions, such as the form of the composition of the present invention.

The composition of the present invention may be in a solid form, e.g., in the form of powder, flake, tablet, or the like. The composition of the present invention may be, especially, a powder form. For example, when at least the solidification-preventing effect is desired to be obtained, the composition of the present invention may be a powder form.

The contents and content ratios of the ingredients contained in the composition of the present invention (i.e., active ingredients and optional additional ingredients) are not particularly restricted as long as the stability-improving effect is obtained. The contents and content ratios of the ingredients in the composition of the present invention can be appropriately set according to various conditions, such as the type of animal cells and the manner in which the composition of the present invention is used.

The total content of the active ingredients in the composition of the present invention is higher than 0% (w/w) and not higher than 100% (w/w). The total content of the active ingredients in the composition of the invention may be, for example, 1% (w/w) or higher, 2% (w/w) or higher, 5% (w/w) or higher, 10% (w/w) or higher, 20% (w/w) or higher, 30% (w/w) or higher, 50% (w/w) or higher, or 70% (w/w) or higher, and 100% (w/w) or lower, 99.9% (w/w) or lower, 90% (w/w) or lower, 70% (w/w) or lower, 50% (w/w) or lower, 30% (w/w) or lower, 20% (w/w) or lower, 10% (w/w) or lower, or 5% (w/w) or lower, or in any range defined by a non-conflicting combination of these exemplary minimum and maximum contents. The total content of the active ingredients in the composition of the present invention may specifically be, for example, 1 to 10% (w/w), 10 to 30% (w/w), 30 to 50% (w/w), 50 to 70% (w/w), 70 to 90% (w/w), or 70 to 100% (w/w). The total content of the active ingredients in the composition of the present invention may specifically be, for example, 1 to 100% (w/w), 5 to 90% (w/w), or 10 to 70% (w/w).

The content of α-ketoglutaric acid in the composition of the present invention may be, for example, 0.5 time or more, 1 time or more, 1.5 times or more, 2 times or more, 2.5 times or more, 3 times or more, 3.5 times or more, 4 times or more, 4.5 times or more, 5 times or more, 5.5 times or more, 6 times or more, 6.5 times or more, 7 times or more, 7.5 times or more, 8 times or more, 8.5 times or more, 9 times or more, 9.5 times or more, or 10 times or more, and 20 times or less, 15 times or less, 12 times or less, 10 times or less, 9.5 times or less, 9 times or less, 8.5 times or less, 8 times or less, 7.5 times or less, 7 times or less, 6.5 times or less, 6 times or less, 5.5 times, 5 times or less, 4.5 times or less, 4 times or less, 3.5 times or less, 3 times or less, 2.5 times or less, or 2 times or less of the content of the amino acid source (e.g., cysteine) in the composition of the present invention in terms of molar ratio, or in any range defined by a non-conflicting combination of these exemplary minimum and maximum contents. The content of α-ketoglutaric acid in the composition of the present invention may specifically be, for example, 0.5 to 2 times, 1 to 2 times, 1.5 to 2 times, 2 to 2.5 times, 2.5 to 3 times, 3 to 3.5 times, 3.5 to 4 times, 4 to 4.5 times, 4.5 to 5 times, 5 to 5.5 times, 5.5 to 6 times, 6 to 6.5 times, 6.5 to 7 times, 7 to 7.5 times, 7.5 to 8 times, 8 to 8.5 times, 8.5 to 9 times, 9 to 9.5 times, 9.5 to 10 times, 10 to 12 times, 10 to 15 times, or 10 to 20 times of the content of the amino acid source in the composition of the present invention (e.g., cysteine) in terms of molar ratio. The content of α-ketoglutaric acid in the composition of the present invention may specifically be, for example, 1 to 20 times, 2 to 15 times, 3 to 10 times, or 4 to 7 times of the content of the amino acid source (e.g., cysteine) in the composition of the present invention in terms of molar ratio. When two or more types of amino acid sources are used as the active ingredients, the "content of amino acid source" referred to here means the total content of those amino acid sources. However, when two or more types of amino acid sources are used as the active ingredients, the content of α-ketoglutaric acid in the composition of the present invention may be independently set within any of the ranges of the content of α-ketoglutaric acid exemplified above for the content of each of those amino acid sources.

The content of each active ingredient in the composition of the present invention can be set, for example, to obtain the total content of the active ingredients and the content ratio of the of the active ingredients in the composition of the present invention described above.

The content of each ingredient (i.e., each of the active ingredients and optional additional ingredients) in the composition of the present invention can be set, for example, to obtain the concentration of each ingredient in the medium used in the method of the present invention described later.

The active ingredients are contained in the composition of the present invention in a state that they are mixed with each other. The additional ingredient may be contained in the composition of the present invention in a state that it is mixed with the active ingredients, or may be contained in the composition of the present invention separately from the active ingredients. If the composition of the present invention contains two or more types of additional ingredients, those additional ingredients may be contained in the composition of the present invention in a state that they are mixed together, separately from each other or one another, or separately as any combinations thereof. For example, the composition of the present invention may be provided as a set of a package of the active ingredients and a package of the additional ingredient. In such a case, the ingredients included in the set can be used together at the time of use as appropriate. The composition of the present invention may also be provided in a form in which a hygroscopic agent is enclosed within the package (specifically, at least within the package of the active ingredients). The composition of the present invention (specifically, at least the active ingredients) may also be provided in a form sealed in a packaging material having a moisture-absorbing function.

If a material containing the active ingredients is used, the amount (e.g., content (concentration) or amount to be used) of the active ingredients shall be calculated on the basis of the amount of the active ingredients themselves in the material. In the case where the active ingredients form a salt or hydrate, the amount (e.g., content (concentration) or amount to be used) of the active ingredients shall be calculated on the basis of the value obtained by converting the mass of the salt or hydrate to the mass of equimolar amount of the compound in the free form of the salt or hydrate.

### <2> Method of the present invention

The method of the present invention is a method comprising using the composition of the present invention.

The composition of the present invention can be used for, for example, culturing animal cells. That is, the method of the present invention may be a method for culturing animal cells, which comprises culturing animal cells using the composition of the present invention.

In one embodiment, a target substance may be produced by culturing animal cells. That is, if animal cells having an ability to produce a target substance are used, the target substance can be produced by culturing the cells. In other words, one embodiment of the method of the present invention (specifically, a method for culturing animal cells) may be a method for producing a target substance, which comprises culturing animal cells having an ability to produce the target substance using the composition of the present invention, and collecting the target substance.

The target substance is not particularly restricted so long as it can be produced by animal cells. Examples of the target substance include proteins and viruses. A protein produced as the target substance is also referred to as "target protein". A virus produced as the target substance is also referred to as "target virus".

The animal cells are not particularly restricted. The animal cells can be appropriately selected depending on various conditions, such as, for example, the intended use of the animal cells. For example, when the animal cells are used for production of a target protein, the animal cells are not particularly restricted so long as they can express the target protein. The animal cells are also referred to as "host", "expression host", or "host cells". Examples of the animal include mammals, birds, amphibians, and insects. Examples of the animal include, especially, mammals. Examples of the mammals include rodents, primates, and various other mammals. Examples of the rodents include hamster, mouse, rat, and guinea pig. Examples of the hamster include Chinese hamster. Examples of the primates include human, monkey, and chimpanzee. Examples of the monkey include African green monkey. Other examples of mammals include dog. Examples of the birds include chicken. Examples of the amphibians include African clawed frog. Examples of the insects include fall armyworm (*Spodoptera frugiperda*). Examples of tissue or cells from which the animal cells are derived are not particularly restricted. Examples of tissue or cells from which animal cells are derived include ovary, kidney, adrenal gland, tongue epithelium, olfactory epithelium, pineal gland, thyroid gland, melanocyte, skin, spleen, liver, lung, pancreas, uterus, stomach, colon, small intestine, large intestine, bladder, prostate, testis, thymus gland, muscle, connective tissue, bone, cartilage, vascular tissue, blood (including cord blood), bone marrow, heart, eye, brain, and nerve tissue. The animal cells may have or may have not differentiated. Specific examples of the animal cells include germ cells, somatic cells, stem cells, and progenitor cells. Examples of germ cells include sperm and ovum. Examples of the somatic cells include fibroblasts, bone marrow cells, B lymphocytes, T lymphocytes, neutrophils, erythrocytes, platelets, macrophages, monocytes, osteocytes, pericytes, dendritic cells, adipocytes, mesenchymal cells, epithelial cells, epidermal cells (e.g., keratinocytes, corneocytes etc.), endothelial cells, vascular endothelial cells, liver parenchymal cells, chondrocytes, cumulus cells, nerve cells, glial cells, oligodendrocytes, microglia, astrocytes, cardiac cells, esophageal cells, muscle cells (e.g., smooth muscle cells and skeletal muscle cells), pancreatic beta cells, melanocytes, and mononuclear cells. Examples of the stem cells include adult stem cells such as hematopoietic stem cells, satellite cells, neural stem cells, mesenchymal stem cells, mammary stem cells, olfactory mucosa stem cells, neural crest stem cells, hepatic stem cells, pancreatic stem cells, muscle stem cells, germline stem cells, intestinal stem cells, and hair follicle stem cells; pluripotent stem cells such as embryonic stem cells (ES cells), embryonic tumor cells, embryonic germline stem cells, and induced pluripotent stem cells (iPS cells); and cancer stem cells. Examples of the progenitor cells include satellite cells, pancreatic progenitor cells, vascular progenitor cells, vascular endothelial progenitor cells, and hematopoietic progenitor cells (such as CD34-positive cells derived from umbilical cord blood). Examples of Chinese hamster cells include those of Chinese hamster ovary-derived cell lines (CHO). Specific examples of CHO include CHO-DG44, CHO-K1, CHO DUX (DHFR-), CHO-S, and CHO-MK. Examples of the mouse cells include NS0 cells derived from mouse myeloma. Examples of the human cells include those of human embryonic kidney cell-derived cell lines (HEK). Specific examples of HEK include HEK293 and HEK293T. Examples of the canine cells include MDCK cells derived from canine kidney. Examples of African green monkey cells include those of African green monkey kidney cell-derived cell line (COS). Specific examples of COS include COS-1. Examples of the African clawed frog cells include African clawed frog oocytes. Examples of the cells of fall armyworm include Sf9 cells, Sf21 cells, and SF+ cells derived from ovary of fall armyworm.

The term "animal cell having a target substance-producing ability" means an animal cell that has an ability to produce a target substance. The term "animal cell having an ability to produce a target substance" may specifically mean an animal cell that has an ability to produce a target substance (e.g., express a target protein) when cultured in a culture medium and to accumulate the target substance in the culture medium to such an extent that the target substance can be collected. The expression "accumulate in the culture medium" may specifically mean to accumulate in the culture medium, cell surface layers, inside of cells, or a combination thereof. When the target substance accumulates outside the cells (e.g., in the medium or on the cell surfaces), the production is also referred to as "secretion" or "secretory production" of the target substance. In other words, the animal cells may have a secretory production ability for a target substance (ability to produce and secrete a target substance). The accumulation amount of the target substance may be, for example, 10 µg/L or more, 1 mg/L or more, 100 mg/L or more, or 1 g/L or more in terms of the amount of accumulation in the culture. The animal cells may have an ability to produce one type of target substance, or may have an ability to produce two or more types of target substances.

The animal cells may be those inherently having an ability to produce a target substance or may be those modified to have an ability to produce a target substance. The animal cells may also be those modified so that their inherent ability to produce a target substance is enhanced. The animal cells having an ability to produce a target substance can be obtained by, for example, imparting an ability to produce a target substance to such animal cells as described above, or by enhancing an ability to produce a target substance of such animal cells as described above. For example, an ability to produce a target protein can be conferred or enhanced by introducing a gene encoding the target protein. A gene encoding a target protein is also referred to as a "target protein gene". Further, for example, an ability to produce a target virus can be conferred by infecting animal cells with the target virus.

The target protein is not particularly restricted so long as it can be expressed in animal cells as hosts. The protein can be a protein derived from a host or a heterologous protein. The "heterologous protein" is a protein that is exogenous to the host that produces it (i.e., an animal cell having an ability to produce the target protein). For example, the target protein may be a naturally occurring protein, a modified protein thereof, or a protein with an artificially designed amino acid sequence. The target protein may be, for example, a protein of microbial origin, a protein of plant origin, a protein of animal origin, or a protein of viral origin. The target protein may be, especially, a protein of human origin. The target protein may be a monomeric protein or a multimeric protein. The target protein may be a secretary protein or a non-secretary protein. The term "protein" also encompasses those called peptides, such as oligopeptides and polypeptides.

Specific examples of the target protein include enzymes, physiologically active proteins, receptor proteins, antigenic proteins, and any other proteins.

Examples of the enzymes include cellulase, xylanase, transglutaminase, protein-glutaminase, protein-asparaginase, isomaltodextranase, protease, endopeptidase, exopeptidase, aminopeptidase, carboxypeptidase, collagenase, chitinase, γ-glutamylvaline synthase, glutamate-cysteine ligase, and glutathione synthetase.

Examples of the physiologically active proteins include growth factors, hormones, cytokines, antibody-related molecules, and antibody mimetics.

Examples of the growth factors include epidermal growth factor (EGF), insulin-like growth factor-1 (IGF-1), transforming growth factor (TGF), nerve growth factor (NGF), brain-derived neurotrophic factor (BDNF), vascular endothelial growth factor (VEGF), granulocyte-colony stimulating factor (G-CSF), granulocyte-macrophage-colony stimulating factor (GM-CSF), platelet-derived growth factor (PDGF), erythropoietin (EPO), thrombopoietin (TPO), acidic fibroblast growth factor (aFGF or FGF1), basic fibroblast growth factor (bFGF or FGF2), fibroblast growth factor (FGF-4), keratinocyte growth factor (KGF-1 or FGF7, and KGF-2 or FGF10), hepatocyte growth factor (HGF), stem cell factor (SCF ), and activin. Examples of activin include activins A, C, and E.

Examples of the hormones include insulin, glucagon, somatostatin, human growth hormone (hGH), parathyroid hormone (PTH), calcitonin, and exenatide.

Examples of the cytokines include interleukins, interferons, and tumor necrosis factor (TNF).

The physiologically active proteins may be an entire protein or a part thereof. Examples of the part of a protein include, for example, a physiologically active part. Specific examples of the physiologically active part include, for example, the physiologically active peptide, teriparatide, which consists of the N-terminus 34 amino acid residues of the mature parathyroid hormone (PTH).

The term "antibody-related molecule" may refer to a protein that contains a molecular species consisting of a single domain or a combination of two or more domains selected from domains constituting a complete antibody. Examples of the domains constituting a complete antibody include the domains of the heavy chain, VH, CH1, CH2, and CH3, and the domains of the light chain, VL and CL. The antibody-related molecules may be a monomeric or multimeric protein as long as they contain the molecular species described above. If the antibody-related molecules are a multimeric protein, they may be a homomultimer consisting of a single type of subunits or a heteromultimer consisting of two or more types of subunits. Specific examples of the antibody-related molecules include complete antibodies, Fab, F(ab'), F(ab')₂ , Fc, dimer consisting of heavy chain (H chain) and light chain (L chain), Fc fusion protein, heavy chain (H chain), light chain (L chain), single chain Fv (scFv), sc(Fv)₂, disulfide-linked Fv (sdFv), diabody, and VHH fragment (Nanobody (registered trademark)). More specific examples of the antibody-related molecules include trastuzumab, adalimumab, nivolumab, VHH antibody N15, and VHH antibody 9g8. Examples of the Fc fusion protein include fusion proteins of various target proteins exemplified in this description and the Fc region. Specific examples of the Fc fusion protein include fusion proteins of Notch ligand and Fc region described later. Examples of the fusion proteins of Notch ligand and Fc region include DLL4-Fc (namely, the fusion protein of DLL4 and Fc region).

The term "antibody mimetic" may refer to an organic compound that can specifically bind to an antigen but is not structurally related to an antibody. Specific examples of the antibody mimetics include the Z domain of protein A (Affibody). More specific examples of the antibody mimetics include ZHER2 Affibody.

Examples of the receptor proteins include receptors for physiologically active proteins and other physiologically active substances. Examples of such other physiologically active substances include neurotransmitters such as dopamine. The receptor proteins may be an orphan receptor for which corresponding ligand is not known.

The antigenic proteins are not particularly restricted so long as they can elicit an immune response. The antigenic protein can be appropriately selected according to, for example, the target of the intended immune response. The antigenic proteins can be used, for example, as vaccines.

Examples of the other proteins include liver-type fatty acid-binding protein (LFABP), fluorescent proteins, immunoglobulin-binding proteins, albumins, fibroin-like proteins, and extracellular proteins. Examples of the fluorescent proteins include green fluorescent protein (GFP). Examples of the immunoglobulin-binding proteins include protein A, protein G, and protein L. Examples of the albumins include human serum albumin. Examples of the Notch ligands include DLL1, DLL3, DLL4, Jagged-1, and Jagged-2. The Notch ligands may be constituted as, for example, a fusion protein with the Fc region. Examples of the fibroin-like proteins include those disclosed in WO2017/090665 and WO2017/171001.

Examples of the extracellular proteins include fibronectin, vitronectin, collagen, osteopontin, laminin, and their partial sequences. Laminin is a protein having a heterotrimeric structure consisting of the α-chain, β-chain, and γ-chain. Examples of laminin include mammalian laminin. Examples of the subunit chains of laminin (i.e., α-chain, β-chain, and γ-chain) include 5 types of α-chains (α1 to α5), three types of β-chains (β1 to β3), and three types of γ-chains (γ1 to γ3). Combinations of these subunit chains constitute various isoforms of laminin. Specifically, examples of laminin include, for example, laminin 111, laminin 121, laminin 211, laminin 213, laminin 221, laminin 311, laminin 321, laminin 332, laminin 411, laminin 421, laminin 423, laminin 511, laminin 521, and laminin 523. Examples of partial sequence of laminin include laminin E8, which is E8 fragment of laminin. The laminin E8 is specifically a protein having a heterotrimeric structure consisting of E8 fragment of the α-chain (α-chain E8), E8 fragment of the β-chain (β-chain E8), and E8 fragment of the γ-chain (γ-chain E8). The subunit chains of laminin E8 (i.e., α-chain E8, β-chain E8, and γ-chain E8) are also collectively referred to as "E8 subunit chains". Examples of the E8 subunit chains include E8 fragments of the laminin subunit chains exemplified above. Laminin E8 presents as various isoforms consisting of combinations of these E8 subunit chains. Specific examples of laminin E8 include laminin 111E8, laminin 121E8, laminin 211E8, laminin 221E8, laminin 332E8, laminin 421E8, laminin 411E8, laminin 511E8, and laminin 521E8. The numbers in the names of the laminin E8 indicate, from left to right, the types of α-chain, β-chain, and γ-chain. That is, for example, "laminin 511E8" means the E8 fragment of laminin 511, specifically, a protein having a heterotrimeric structure consisting of the E8 fragment of the α5-chain (α5-chain E8), E8 fragment of the β1-chain (β1-chain E8), and E8 fragment of the γ1-chain (γ1 chain E8).

The term "α-chain E8" may mean a region near the C-terminus of the α-chain, specifically, the C-terminus fragment of the α-chain excluding the globular domains 4 and 5, more specifically, a fragment consisting of the C-terminus 780 to 830 (e.g., 790 to 800) amino acid residues of the α-chain excluding the globular domains 4 and 5. The term "β-chain E8" may mean a C-terminus fragment of the β-chain, more specifically, a fragment consisting of C-terminus 220 to 230 amino acid residues of the β-chain. The term "γ-chain E8" may mean a C-terminus fragment of the γ-chain, specifically, a fragment consisting of C-terminus 240 to 250 amino acid residues of the γ-chain. Examples of the human α5-chain E8 include the site of the amino acid numbers 2534 to 3327 of GenBank Accession No. NP_005551. Examples of the human β1-chain E8 include the site of the amino acid numbers 1561 to 1786 of GenBank Accession No. NP_002282. Examples of the human γ1-chain E8 include the site of the amino acid numbers 1364 to 1609 of GenBank Accession No. NP_002284.

The target protein may be, for example, a protein having a known or natural amino acid sequence of any of such proteins as described above. The target protein may also be, for example, a variant of a protein having a known or natural amino acid sequence of any of such proteins as described above. Examples of such a variant include proteins having an amino acid sequence derived from a known or natural amino acid sequence by substitution, deletion, insertion or addition of one or several amino acids at one or more positions. The term "one or several" may specifically mean, for example, 1 to 50, 1 to 40, or 1 to 30, preferably 1 to 20, more preferably 1 to 10, further preferably 1 to 5, and especially preferably 1 to 3. Examples of the variant also include proteins having an identity of, for example, 50% or more, 65% or more, or 80% or more, preferably 90% or more, more preferably 95% or more, further preferably 97% or more, especially preferably 99% or more, to corresponding entire known or natural amino acid sequences. Proteins identified with a species of origin are not limited to proteins found in that species itself, but also includes proteins having the amino acid sequences of the proteins found in the species and their variants. The variants may or may not be found in the species. That is, for example, "human-derived proteins" are not limited to proteins found in humans per se, but shall include proteins having the amino acid sequences of the proteins found in humans and variants thereof.

The "identity" between amino acid sequences means identity between amino acid sequences calculated with blastp using the default settings of Scoring Parameters (Matrix, BLOSUM62; Gap Costs, Existence=11 and Extension=1; and Compositional Adjustments, Conditional compositional score matrix adjustment).

The target protein gene is not particularly restricted as long as it encodes such a target protein as described above. The target protein gene may be, for example, a gene having a known or natural nucleotide sequence of a gene encoding any of such proteins as described above. The target protein gene may also be, for example, a variant of a gene having a known or natural nucleotide sequence of a gene encoding any of such proteins as described above. The target protein gene may be, for example, modified to encode a protein having such a variant sequence as exemplified above. The target protein gene may include replacement of a codon with an equivalent codon. The target protein gene may be, for example, modified to have codons optimized according to frequencies of codons found in the host cells.

In the present invention, the term "gene" is not limited to DNA, but may encompass any polynucleotide as long as it encodes a corresponding expression product. In other words, the term "target protein gene" may mean any polynucleotide that encodes a target protein. The target protein gene may be DNA, RNA, or a combination thereof. The target protein gene may be single-stranded or double-stranded. The target protein gene may be single-stranded DNA or single-stranded RNA. The target protein gene may be double-stranded DNA, double-stranded RNA, or a hybrid strand consisting of DNA and RNA strands. The target protein gene may contain both DNA and RNA residues in a single polynucleotide strand. The target protein gene may or may not contain an intron. The type of the target protein gene may be appropriately selected according to various conditions, such as the means for expressing the target protein.

The expression "having an (amino acid or nucleotide) sequence" means "containing the (amino acid or nucleotide) sequence" and also means "consisting of the (amino acid or nucleotide) sequence", unless otherwise specified.

The target protein is expressed from the target protein gene. That is, animal cells having an ability to produce the target protein have the target protein gene. Specifically, the animal cells having a target protein-producing ability have the target protein gene in such a manner that the target protein gene can be expressed. It is sufficient that the animal cells having a target protein-producing ability have the target protein gene until the target protein is expressed to a desired degree. In other words, the animal cells having a target protein-producing ability may or may not have the target protein gene after expression of the target protein. The terms "expression of a target protein gene" and "expression of a target protein" may be synonymously used.

The target protein gene can be obtained by cloning from an organism that has the target protein gene. Nucleic acids such as genomic DNA or cDNA containing the gene can be used for cloning. The target protein gene can also be obtained by chemical synthesis (Gene, 60(1), 115-127 (1987)).

The obtained target protein gene can be used as it is or after appropriate modification. In other words, variants of the target protein gene can be obtained by modifying it. The gene can be modified by known methods. For example, a site-specific mutagenesis can be used to introduce a desired mutation at a target site of DNA. Examples of the site-specific mutagenesis include methods using PCR (Higuchi, R., 61, in PCR technology, Erlich, H. A. Eds., Stockton press (1989); and Carter, P., Meth. in Enzymol., 154, 382 (1987), and methods using phages (Kramer, W. and Frits, H. J., Meth. in Enzymol., 154, 350 (1987); and Kunkel, T. A. et al., Meth. in Enzymol., 154, 367 (1987)). Variants of the target protein gene may also be directly obtained by chemical synthesis.

The mode of introducing the target protein gene into the host cell is not particularly restricted. The target protein gene should be retained in the host cell so that it can be expressed. Specifically, for example, when a target protein gene is introduced in a form requiring transcription such as DNA, the target protein gene may be maintained in the host cell so that it can be expressed under the control of a promoter that functions in the host cell. In the host cell, the target protein gene may be present outside the chromosome or introduced into the chromosome. When two or more types of genes are introduced, it is sufficient that each gene is retained in the host cell so that it can be expressed.

The promoter for expressing the target protein gene is not particularly restricted as long as it functions in the host cell. The term "promoter that functions in the host cell" means a promoter that shows a promoter activity in the host cell. The promoter may be a promoter derived from the host cell or a promoter of a heterologous origin. The promoter may be a promoter native to the target protein gene or a promoter of another gene. The promoter may be a promoter that is stronger than the promoter native to the target protein gene. Examples of promoters that function in animal cells include the SV40 promoter, EF1a promoter, RSV promoter, CMV promoter, and SRalpha promoter. In addition, highly active forms of conventional promoters may be obtained by using various reporter genes and used as the promoter. Methods for evaluating strength of promoters and examples of strong promoters are described in the paper of Goldstein et al. (Prokaryotic promoters in biotechnology. Biotechnol. Annu. Rev., 1, 105-128 (1995)), and so forth.

The target protein gene can be introduced into the host cell by using, for example, a vector containing the gene. A vector containing a target protein gene is also referred to as "expression vector of a target protein gene". An expression vector of a target protein gene can be constructed by, for example, ligating a DNA fragment containing the target protein gene with a vector. By introducing an expression vector of a target protein gene into a host cell, the gene can be introduced into the host cell. The vector may have a marker such as a drug resistance gene. The vector may also have an expression regulatory sequence such as a promoter for expressing the inserted gene. The vector can be selected depending on various conditions, such as the type of host cell and the mode of the introduction of the target protein gene. Examples of vectors that can be used for gene transfer into animal cells include plasmid vectors and viral vectors. Examples of viral vectors include, for example, retrovirus vectors and adenovirus vectors. Examples of plasmid vectors include, for example, pcDNA series vectors (pcDNA3.1, etc., Thermo Fisher Scientific), pBApo-CMV series vectors (Takara Bio), and pCI-neo (Promega). Depending on the type and configuration of the vector, the vector can be incorporated into the host cell chromosome, autonomously replicate outside the host cell chromosome, or be temporarily retained outside the host cell chromosome. For example, vectors having a viral replication origin such as SV40 replication origin can autonomously replicate outside the chromosomes of animal cells. Specifically, for example, the pcDNA series vectors have the SV40 replication origin and can autonomously replicate outside the chromosome in host cells that express the SV40 large T antigen (such as COS-1 and HEK293T).

The target protein gene can also be introduced into a host cell by, for example, introducing a nucleic acid fragment containing the gene into the host cell. Examples of such a nucleic acid fragment include linear DNA and linear RNA. Examples of linear RNA include mRNA and cRNA.

The method for introducing a nucleic acid such as vectors and nucleic acid fragments into a host cell can be selected according to various conditions such as the type of host cell. Examples of the method for introducing a nucleic acid such as vectors and nucleic acid fragments into animal cells include the DEAE-dextran method, calcium phosphate method, lipofection method, electroporation method, and microinjection method. If the vector is a viral vector, it can be introduced into the host cell by infecting the host cell with the vector (virus).

In addition, cells that inherently have the target protein gene may be modified so that the expression of the target protein gene increases, and then used. The expression that "expression of the target protein gene increases" means that the expression of the gene per cell increases compared with unmodified cells. The term "unmodified cells" used here means control cells that have not been modified so that the expression of the target gene increases. Examples of the unmodified cells include wild-type cells and original cells to which the modification is to be added. Examples of the means for increasing expression of a target protein gene include increasing copy number of the target protein gene and improving transcription efficiency or translation efficiency of the target protein gene. Increasing copy number of a target protein gene can be achieved by introducing the target protein gene into the host cell. Introduction of the target protein gene can be carried out as described above. The target protein gene to be introduced may be derived from the host cell or of a heterologous origin. Improvement of transcription or translation efficiency of a target protein gene can be achieved by modifying an expression regulatory sequence of the gene, such as a promoter. For example, improvement of transcription efficiency of a target protein gene can be achieved by replacing the promoter of the target protein gene with a stronger promoter.

In the method for culturing animal cells, the composition of the present invention is used to culture animal cells. That is, the culture of animal cells is carried out by using the composition of the present invention. For example, the composition of the present invention may be used for culturing animal cells as a culture medium. That is, for example, when the composition of the present invention is a culture medium, the animal cells may be cultured in the composition of the present invention (i.e., culture medium). In other words, the expression "culturing animal cells by using the composition of the present invention" may mean, for example, culturing animal cells in the composition of the present invention that is a culture medium. The composition of the present invention may also be used for culturing animal cells as, for example, a medium additive. That is, for example, when the composition of the present invention is a medium additive, the composition of the present invention (i.e., medium additive) may be added to the medium, and the animal cells may be cultured in the medium to which the composition of the present invention has been added. In other words, "culturing animal cells by using the composition of the present invention" may mean, for example, adding the composition of the present invention, which is a medium additive, to a medium and culturing the animal cells in the medium to which the composition of the present invention has been added.

The term "culture of animal cells" is not limited to those aiming at proliferation of animal cells, but may also encompass those not aiming at proliferation of animal cells, such as those aiming at maintenance of animal cells or production of target substances by animal cells.

The medium composition and culture conditions are not particularly restricted as long as the purpose of culturing animal cells can be achieved, except that the culture is carried out by using the composition of the present invention. For example, when the purpose is to proliferate animal cells, the medium composition and culture conditions can be configured so that the animal cells proliferate. For example, when the purpose is to maintain animal cells, the medium composition and culture conditions can be configured so that animal cells are maintained (i.e., the animal cells survive). Further, for example, when the purpose is to produce a target substance such as a target protein, the medium composition and culture conditions can be configured so that the target substance is produced (e.g., so that the target protein is expressed, if the target substance is a target protein). When the purpose is not to proliferate animal cells, the animal cells may or may not proliferate during the culture. Even when proliferation of animal cells is not intended, typically, animal cells may proliferate during culture. The medium composition and culture conditions may be appropriately set according to various conditions, for example, the type of animal cells. The culture can be carried out by, for example, using a usual medium and usual conditions used for culturing animal cells as they are or with appropriate modifications, except that the culture is carried out by using the composition of the present invention.

The culture may be carried out as seed culture and main culture, which are separately performed. If the culture is carried out as seed culture and main culture separately performed, the composition of the present invention may be used in either or both the seed culture and the main culture. If the culture is carried out as seed culture and main culture separately performed, the composition of the present invention may be used, especially, at least in the main culture. If the culture is carried out as seed culture and main culture separately performed, the descriptions for the culture (e.g., "culture period (duration of culture)" or "start of culture") may be applied to both the seed culture and the main culture, unless otherwise noted. The culture conditions for the seed culture and the main culture may or may not be the same. When the purpose is to produce a target substance such as a target protein, it is sufficient that the target substance is produced at least during the period of the main culture. For example, after the animal cells are sufficiently proliferated by seed culture, the target substance such as a target protein may be produced by the main culture.

The culture may be performed as batch culture, fed-batch culture, continuous culture, or a combination thereof. Examples of the continuous culture include perfusion culture and chemostat culture. The culture medium used at the start of culture is also referred to as "starting medium" or "basal medium". The medium supplied to the culture system (e.g., starting medium) in the fed-batch culture is also referred to as "feed medium". The medium supplied to the culture system (e.g., starting medium) in continuous culture (which is not limited to perfusion culture) is also referred to as "perfusion medium". Supplying feed medium or perfusion medium to a culture system in the fed-batch culture or continuous culture is also referred to simply as "medium supply. Medium supply may be performed throughout the entire culture period or during only a part of the culture period. The medium supply may be performed continuously or intermittently. During the culture (especially continuous culture such as perfusion culture), the culture medium may be withdrawn. The culture medium may be withdrawn throughout the entire period of the culture or during only a part of the culture period. Culture medium may be withdrawn continuously or intermittently. Withdrawal of the culture medium and medium supply may or may not be performed simultaneously. When the culture is performed by seed culture and main culture, which are separately performed, the culture modes of the seed culture and the main culture may or may not be the same.

The culture can be performed by using, for example, a liquid medium.

The medium used for the culture can be selected, for example, independently for the basal medium, feed medium, and perfusion medium.

The medium used for the culture may be a commercially available medium or a medium appropriately prepared. Examples of commercially available media include culture media for animal cell culture such as D-MEM (Dulbecco's Modified Eagle Medium), CELLiST Basal Media BASAL3, BASAL4P, and BASAL10 (Ajinomoto Co., Ltd.), Opti-MEM (Thermo Fisher Scientific), RPMI 1640 (Thermo Fisher Scientific), CD293 (Thermo Fisher Scientific), CHO-S-SFMII (Thermo Fisher Scientific), CHO-SF (Sigma-Aldrich), EX-CELL CD CHO (Sigma-Aldrich), EX-CELLTM302 (Sigma-Aldrich), IS CHO-CD (Irvine Scientific), and IS CHO-CDXP (Irvine Scientific).

The culture medium used for the culture may be, for example, the composition of the present invention (specifically, the composition of the present invention that is a culture medium). That is, when the composition of the present invention is a culture medium, the composition of the present invention may be used for the culture as it is or as an appropriately prepared liquid medium having a desired composition. For example, a liquid medium may be prepared by diluting the composition of the present invention with an aqueous medium such as water or aqueous buffer solution, and then used for the culture. The composition of the present invention may be used as one or more types of media selected from the basal medium, feed medium, and perfusion medium. That is, for example, in the case of fed-batch culture, the composition of the present invention may be used as one or both of the basal medium and feed medium. In the case of continuous culture, for example, the composition of the present invention may be used as one or both of the basal medium and perfusion medium.

The medium used for the culture may be, for example, a medium to which the composition of the present invention (specifically, the composition of the present invention that is a medium additive) has been added. The culture medium to which the composition of the present invention is to be added may be a commercially available culture medium or a culture medium appropriately prepared. The composition of the present invention may be used by adding it to one or more types of media selected from the basal medium, feed medium, and perfusion medium. That is, for example, in the case of fed-batch culture, the composition of the present invention may be used by adding it to one or both of the basal medium and feed medium. In the case of continuous culture, for example, the composition of the present invention may be used by adding it to one or both of the basal medium and perfusion medium.

The medium used for the culture may contain, for example, an amino acid source and/or α-ketoglutaric acid. For example, when the medium used for the culture is the composition of the present invention, or when the medium used for the culture is a medium to which the composition of the present invention has been added, the medium used for the culture contains an amino acid source and α-ketoglutaric acid. The medium used for the culture may also contain, for example, various medium components. The culture medium components are as described above in the explanation of the composition of the present invention. The culture medium exemplified above may be used for the culture after adding, for example, an amino acid source, α-ketoglutaric acid, various culture medium components, and other components as appropriate.

Various components such as the amino acid source, α-ketoglutaric acid, and culture medium components may all be contained in the starting medium, feed medium, perfusion medium, or a combination thereof. In other words, various components such the amino acid source etc. may be supplied to the medium independently or in any combination thereof during the process of the culture. Any of these components may be supplied once or multiple times or continuously. The compositions (e.g., types and/or concentrations of components contained) of the starting medium, feed medium, and perfusion medium may or may not be the same. That is, the types of components contained in the starting medium may or may not be the same as the types of components contained in the feed medium or perfusion medium. The concentration of each component in the starting medium may or may not be the same as the concentration of that component in the feed medium or perfusion medium. For example, if a feed medium is used for the perfusion culture, the compositions of the starting medium and the feed medium may be the same. Two or more types of feed media or perfusion media having different compositions (e.g., types and/or concentrations of components contained) may also be used. For example, feed medium or perfusion medium is supplied multiple times or supplied intermittently, the compositions of the feed media or perfusion media for the respective times may or may not be the same. In addition, the various components, such as amino acid source, may be supplied to the medium in a form that is not contained in the feed medium or perfusion medium, such as in the form of powder. For example, any of the various components, such as amino acid source, may be supplied to the medium by adding the composition of the present invention.

The inoculation amount of animal cells at the start of culture may be, for example, 1 × 10³ cells/mL or more, 1 × 10⁴ cells/mL or more, 1 × 10⁵ cells/mL or more, 1 × 10⁶ cells/mL or more, or 1 × 10⁷ cells/mL or more, and 1 × 10⁸ cells/mL or less, 1 × 10⁷ cells/mL or less, 1 × 10⁶ cells/mL or less, 1 × 10⁵ cells/mL or less, or 1 × 10⁴ cells/mL or less, or in any range defined by a non-conflicting combination of these exemplary minimum and maximum amounts. The inoculation amount of animal cells at the start of culture may specifically be, for example, 1 × 10³ to 1 × 10⁴ cells/mL, 1 × 10⁴ to 1 × 10⁵ cells/mL, 1 × 10⁵ to 1 × 10⁶ cells/mL, 1 × 10⁶ to 1 × 10⁷ cells/mL, or 1 × 10⁷ to 1 × 10⁸ cells/mL in terms of viable cell count. The inoculation amount of animal cells at the start of culture may specifically be, for example, 1 × 10³ to 1 × 10⁸ cells/mL, 1 × 10⁴ to 1 × 10⁷ cells/mL, or 1 × 10⁵ to 1 × 10⁶ cells/mL in terms of viable cell count. The viable cell count can be determined by using, for example, the cell viability autoanalyzer, Vi-CELL^{™} XR (Beckman Coulter).

The cultures may be performed, for example, in a CO₂-containing atmosphere such as 5 to 15% CO₂. The pH of the medium may be, for example, around neutral. The term "around neutral" may mean, for example, pH 6 to 8, pH 6.5 to 7.5, or pH 6.8 to 7.2. During the culture, the pH of the medium may be adjusted as required. The pH of the medium can be adjusted by using various alkaline or acidic substances such as ammonia gas, aqueous ammonia, sodium carbonate, sodium bicarbonate, potassium carbonate, potassium bicarbonate, magnesium carbonate, sodium hydroxide, potassium hydroxide, calcium hydroxide, and magnesium hydroxide. The culture temperature may be, for example, 30 to 38°C. The culture period may be, for example, 0.5 day or longer, 1 day or longer, 2 days or longer, 3 days or longer, 4 days or longer, 5 days or longer, 6 days or longer, 7 days or longer, 8 days or longer, 9 days or longer, 10 days or longer, 12 days or longer, 15 days or longer, or 20 days or longer, and 60 days or shorter, 50 days or shorter, 40 days or shorter, 30 days or shorter, 25 days or shorter, 20 days or less, 15 days or less, 12 days or less, 10 days or less, 9 days or less, 8 days or less, or 7 days or less, or in any range defined by a non-conflicting combination of these exemplary minimum and maximum periods. The culture period may be specifically, for example, 1 to 60 days, 3 to 25 days, or 5 to 20 days. During the culture, expression of a gene such as target protein gene may be induced as required.

The amino acid source concentration in the medium may be, for example, 0.1 mM or higher, 0.3 mM or higher, 0.5 mM or higher, 0.7 mM or higher, 1 mM or higher, 1.5 mM or higher, 2 mM or higher, 3 mM or higher, 4 mM or higher, or 5 mM or higher, and 30 mM or lower, 25 mM or lower, 20 mM or lower, 15 mM or lower, 10 mM or lower, 7 mM or lower, 5 mM or lower, 4 mM or lower, 3 mM or lower, 2 mM or lower, 1.5 mM or lower, or 1mM or lower, or in any range defined by a non-conflicting combination of these exemplary minimum and maximum concentrations. The amino acid source concentration in the medium may be specifically, for example, 0.1 to 1 mM, 0.3 to 1 mM, 0.5 to 1 mM, 0.7 to 1 mM, 1 to 1.5 mM, 1.5 to 2 mM, 2 to 3 mM, 3 to 4 mM, 4 to 5 mM, 5 to 7 mM, 5 to 10 mM, 5 to 15 mM, 5 to 20 mM, 5 to 25 mM, or 5 to 30 mM. The amino acid source concentration in the medium may be specifically, for example, 0.1 to 10 mM, 0.3 to 5 mM, 0.5 to 3 mM, or 1 to 2 mM, or 0.1 to 30 mM, 0.3 to 25 mM, or 0.5 to 20 mM. When two or more types of amino acid sources are used as the active ingredients, the term "amino acid source concentration" used here means the total concentration of those amino acid sources. However, when two or more types of amino acid sources are used as the active ingredients, the concentrations of those amino acid sources may also be independently set within the ranges of the amino acid source concentration exemplified above. The description for amino acid source concentrations exemplified above can also be applied to the concentration of amino acid source that is an additional component. Specifically, for example, the cysteine source concentration in the medium may be 0.1 to 10 mM, 0.3 to 5 mM, 0.5 to 3 mM, or 1 to 2 mM. Specifically, for example, the tyrosine source concentration in the medium may be 0.1 to 30 mM, 0.3 to 25 mM, or 0.5 to 20 mM.

The α-ketoglutaric acid concentration in the medium may be, for example, 0.1 mM or higher, 0.3 mM or higher, 0.5 mM or higher, 0.7 mM or higher, 1 mM or higher, 1.5 mM or higher, 2 mM or higher, 3 mM or higher, 4 mM or higher, 5 mM or higher, 7 mM or higher, or 10 mM or higher, and 50 mM or lower, 30 mM or lower, 20 mM or lower, 15 mM or lower, 10 mM or lower, 7 mM or lower, 5 mM or lower, 4 mM or lower, 3 mM or lower, 2 mM or lower, 1.5 mM or lower, or 1 mM or lower, or in any range defined by a non-conflicting combination of these exemplary minimum and maximum concentrations. The α-ketoglutaric acid concentration in the medium may be specifically, for example, 0.1 to 1 mM, 0.3 to 1 mM, 0.5 to 1 mM, 0.7 to 1 mM, 1 to 1.5 mM, 1.5 to 2 mM, 2 to 3 mM, 3 to 4 mM, 4 to 5 mM, 5 to 7 mM, 7 to 10 mM, 10 to 15 mM, 10 to 20 mM, 10 to 30 mM, or 10 to 50 mM. The α-ketoglutaric acid concentration in the medium may be specifically, for example, 0.1 to 30 mM, 0.3 to 20 mM, 0.5 to 15 mM, or 1 to 10 mM.

All of the various components, such as the amino acid source, may be contained in the culture medium during the entire period of the culture, or may be contained in the culture medium during only a part of the culture period. In other words, the expression that "the culture is carried out in a medium containing a certain component" means that it is sufficient if the component is contained in the medium during at least a part of the culture period, and it is not necessary that the component be contained in the medium during the entire period of the culture. All of the various components such as the amino acid source may be contained in the culture medium, for example, at the time of the start of the culture, or supplied to the culture medium after the start of the culture. All of the various components, such as the amino acid source, may be contained in the culture medium, for example, at the time of the start of the the culture, and further supplied to the culture medium after the start of the culture (e.g., after consumption of the active ingredients).

All of the various components, such as the amino acid source, may be contained in the culture medium, for example, at the concentrations exemplified above during the entire period of culture, or contained in the culture medium at the concentrations exemplified above during only a part of the culture period. In other words, the expression that "the culture is performed in a medium containing a certain component at a certain concentration" means that it is sufficient if the concentration of the component in the medium is within the range of the concentration during at least a part of the culture period, and it is not necessary that the concentration of the component in the medium be within the range of the concentration during the entire period of the culture. All of the various components, such as the amino acid source, may be contained in the culture medium, for example, at the concentrations exemplified above at the start of the culture, or may be supplied to the culture medium so as to be at the concentrations exemplified above after the start of the culture. All of the various components, such as the amino acid source, may be contained in the culture medium, for example, at the concentrations exemplified above at the start of the culture, and may be further supplied to the culture medium after the start of the culture (e.g., after consumption of the components) so as to be at the concentrations exemplified above.

The length of the "part of the culture period" is not particularly limited as long as the animal cells can be cultured. The length of the "part of the culture period" can be appropriately set according to various conditions such as the type of component, type of animal cell, length of the culture period, and the desired production amount of the target substance. The "part of the culture period" may be, for example, 10% or more, 20% or more, 30% or more, 40% or more, 50% or more, 60% or more, 70% or more, 80% or more, 90% or more, 95% or more, 97% or more, or 99% or more of the total period of the culture. The "part of the culture period" may also be, for example, 0.5 day or longer, 1 day or longer, 2 days or longer, 3 days or longer, 4 days or longer, 5 days or longer, 6 days or longer, 7 days or longer, 8 days or longer, 9 days or longer, 10 days or longer, 12 days or longer, or 15 days or longer.

The concentrations of the various components such as the amino acid source in the culture medium may all be set at the concentrations exemplified above as, for example, an average value throughout a specific period during the culture. In other words, the expression that "the culture is performed in a medium containing a certain component at a certain concentration" may mean that the average value of the concentration of the component in the medium is within the range of the concentration in a specific period during the culture. The "average value of the concentration of the component in the medium in a specific period during the culture" is not particularly restricted as long as the variation of the concentration of the component during the specific period of the culture can be determined, but may mean, for example, an average of the concentrations of the component in the medium measured every 60 minutes, 30 minutes, 20 minutes, or 10 minutes in the specific period during the culture. The "specific period during the culture" can be the entire period of the culture or a part of the culture period. The "part of the culture period" is as described above.

All of the various components, such as the amino acid source, may be supplied to the culture medium throughout the entire culture period or during only a part of the culture period. The "part of the culture period" is as described above. All of the various components, such as the amino acid source, may be supplied to the culture medium, for example, continuously or intermittently. All of the various components, such as the amino acid source, may be supplied to the culture medium, for example, daily or every few days.

The concentrations of the various components, such as the amino acid source, in the feed medium or perfusion medium may be, for example, in the ranges of the concentrations of the components in the medium exemplified above. The concentrations of the various components, such as amino acid source, in the feed medium or perfusion medium may be, for example, 1 time or higher, 1.1 times or higher, 1.3 times or higher, 1.5 times or higher, 2 times or higher, 3 times or higher, 5 times or higher, 7 times or higher, 10 times or higher, 15 times or higher, or 20 times or higher, and 100 times or lower, 70 times or lower, 50 times or lower, 30 times or lower, 20 times or lower, 15 times or lower, 10 times or lower, 7 times or lower, 5 times or lower, 3 times or lower, or 2 times or lower of the concentrations of the components in the medium exemplified above, or in any range defined by a non-conflicting combination of these exemplary minimum and maximum concentrations. The concentrations of the various components such as the amino acid source in the feed medium or perfusion medium may be specifically, for example, 1 to 2 times, 1.1 to 2 times, 1.3 to 2 times, 1.5 to 2 times, 2 to 3 times, 3 to 5 times, 5 to 7 times, 7 to 10 times, 10 to 15 times, 15 to 20 times, 20 to 30 times, 20 to 50 times, 20 to 70 times, or 20 to 100 times the concentrations of the components in the medium exemplified above. The concentrations of the various components such as the amino acid source in the feed medium or perfusion medium may be, for example, 1 to 100 times, 2 to 50 times, or 5 to 20 times the concentrations of the components in the medium exemplified above. The concentration of the amino acid source (e.g., cysteine concentration) in the feed medium or perfusion medium may be, for example, 1 mM or higher, 2 mM or higher, 5 mM or higher, 10 mM or higher, 15 mM or higher, 20 mM or higher, 30 mM or higher, 40 mM or higher, 50 mM or higher, or 60 mM or higher, and may be the saturation concentration or lower, 100 mM or lower, 90 mM or lower, 80 mM or lower, 70 mM or lower, 60 mM or lower, 50 mM or lower, 40 mM or lower, 30 mM or lower, or 20 mM or lower, or in any range defined by a non-conflicting combination of these exemplary minimum and maximum concentrations. The amino acid source concentration (e.g., cysteine concentration) in the feed medium or perfusion medium may be specifically, for example, 1 to 20 mM, 2 to 20 mM, 5 to 20 mM, 10 to 20 mM, 15 to 20 mM, 20 to 30 mM, 30 to 40 mM, 40 to 50 mM, 50 to 60 mM, 60 to 70 mM, 60 to 80 mM, 60 to 90 mM, 60 to 100 mM, or 60 mM to the saturation concentration. The amino acid source concentration (e.g., cysteine concentration) in the feed medium or perfusion medium may be specifically, for example, 5 to 100 mM, 10 to 90 mM, or 20 to 80 mM. When two or more types of amino acid sources are used as the active ingredients, the "amino acid source concentration" referred to here means the total concentration of those amino acid sources. However, when two or more types of amino acid sources are used as the active ingredients, the concentrations of those amino acid sources may also be independently set within ranges of the amino acid source concentration exemplified above. The descriptions for the amino acid source concentrations exemplified above can also be applied to the concentrations of amino acid source as additional component.

The concentrations of the various components, such as the amino acid source, can all be measured by, for example, known methods used for detection or identification of compounds. Examples of such methods include, for example, HPLC, UPLC, LC/MS, GC/MS, and NMR. These methods can also be used to confirm that the target substance has been produced. One of these methods may be independently used, or two or more may be used in combination as appropriate.

Animal cells can be cultured as described above. When animal cells have a target substance-producing ability such as a target protein-producing ability, the target substance is produced (e.g., target protein is expressed) by culturing the animal cells as described above, and thus a culture containing the target substance can be obtained. The target substance such as a target protein may accumulate, specifically, in the culture medium, cell surface layers, inside of the cells, or a combination thereof.

Hereafter, with reference to, especially, the case of producing a target protein, operations such as confirmation of production, collection, purification, and so forth of a target protein will be explained, but such operations can also be appropriately performed for target substances other than target protein.

Production of a target protein can be confirmed by known methods used for detection or identification of proteins. Examples of such methods include, for example, SDS-PAGE, Western blotting, mass spectrometry, N-terminus amino acid sequence analysis, and enzyme activity assay. One of these methods may be used independently, or two or more may be used in combination as appropriate.

The target protein can be collected as appropriate. Specifically, the target protein can be collected as an appropriate fraction containing the target protein. Examples of such a fraction include, for example, culture, culture supernatant, cultured cells, and processed products of cultured cells (disruption product, lysate, extract (cell-free extract), etc.). The cultured cells may be obtained in the form of, for example, immobilized cells immobilized with carriers such as acrylamide or carrageenan.

The target protein may be further purified to a desired degree.

If the target protein accumulates in the culture medium, for example, after removal of solids such as cells from the culture by centrifugation or the like, the target protein can be purified from the supernatant.

If the target protein accumulates in the inside of the cells, the target protein can be purified from a product of a treatment of the cells such as disruption, lysis, and extraction of the cells. The cells can be collected from the culture by centrifugation or other means. The treatment of the cells such as disruption, lysis, and extraction of the cells can be performed by known methods. Examples of such methods include, for example, ultrasonic disruption, dynomilling, bead disruption, French press disruption, and lysozyme treatment. One of these methods may be used independently, or two or more may be used in combination as appropriate.

If the target protein accumulates in the cell surface layers, the target protein can be obtained by, for example solubilizing the protein and then purified from the solubilization product. Solubilization can be performed by known methods. Examples of such methods include, for example, increasing salt concentration and using surfactants. One of these methods may be used independently, or two or more methods may be used in combination as appropriate.

Purification of the target protein (e.g., purification from such a supernatant, processed product, or solubilization product as described above) can be performed by known methods used for purification of proteins. Examples of such methods include, for example, ammonium sulfate fractionation, ion exchange chromatography, hydrophobic chromatography, affinity chromatography, gel filtration chromatography, and isoelectric precipitation. One of these methods may be used independently or two or more may be used in combination as appropriate.

The target protein may be obtained in a free state or as an immobilized enzyme immobilized on a solid phase such as a resin.

The collected target protein may be formulated in the form of a preparation as appropriate. The form of the preparation is not particularly restricted and can be appropriately set according to various conditions such as the intended use of the target protein. Examples of the form of the preparation include solution, suspension, powder, tablet, pill, and capsule. For the formulation, for example, pharmacologically acceptable additives such as excipient, binder, disintegrant, lubricant, stabilizer, flavor enhancer, odor enhancer, flavoring agent, diluent, and surfactant can be used.

### <4> Use of active ingredients

The present invention also relates to use of the active ingredients in the applications exemplified above. That is, the present invention relates to, for example, use of the active ingredients for improving stability of a composition and use of the active ingredients in production of a composition for animal cell culture. In other words, the present invention relates to a method for realizing the uses exemplified above by using the active ingredients. That is, the present invention relates to, for example, a method for improving stability of a composition, comprising mixing the active ingredients, and a method for producing a composition for animal cell culture, which comprises mixing the active ingredients.

The present invention also relates to an active ingredient for use in the uses exemplified above. That is, the present invention relates to, for example, an active ingredient for use in improving stability of a composition and for use in production of a composition for animal cell culture.

The present invention also relates to use of each active ingredient for use in combination with another active ingredient. Each active ingredient may be used in combination with the other active ingredient for the uses exemplified above. That is, the present invention relates to, for example, use of a salt of α-ketoglutaric acid for improving stability of a composition containing an amino acid source (e.g., cysteine), or use of free L-cysteine anhydrate for improving stability of a composition containing α-ketoglutaric acid. Examples of the use of a salt of α-ketoglutaric acid for improving stability of a composition containing an amino acid source (e.g., cysteine) include, especially, use of a salt of α-ketoglutaric acid for preventing solidification of a composition containing an amino acid source (e.g., cysteine) and use of a salt of α-ketoglutaric acid for preventing decrease in cysteine content in a composition containing cysteine. Examples of the use of free L-cysteine anhydrate for improving stability of a composition containing α-ketoglutaric acid include, especially, use of free L-cysteine anhydrate for preventing solidification of a composition containing α-ketoglutaric acid. In other words, the present invention relates to a method for realizing uses exemplified above by using each active ingredient in combination with the other active ingredient. Namely, the present invention relates to a method for improving stability of a composition containing an amino acid source (e.g., cysteine), comprising, for example, mixing a salt of α-ketoglutaric acid with the composition containing an amino acid source (e.g., cysteine) and a method for improving stability of a composition containing α-ketoglutaric acid, comprising mixing free L-cysteine anhydrate with the composition containing α-ketoglutaric acid. Examples of the method for improving stability of a composition containing an amino acid source (e.g., cysteine) include, especially, a method for preventing solidification of a composition containing an amino acid source (e.g., cysteine) and a method for preventing decrease in cysteine content in a composition containing cysteine. Examples of the method for improving stability of a composition containing α-ketoglutaric acid include, especially, a method for preventing solidification of a composition containing α-ketoglutaric acid.

### Examples

Hereafter, the present invention will be explained more specifically with reference to the following non-limiting examples.

### Example 1: Prevention of solidification of composition

In this example, the effect of change in the forms of L-cysteine and/or α-ketoglutaric acid on conditions of mixed powder of L-cysteine and α-ketoglutaric acid was evaluated.

### (1) Preparation of mixed powders

L-Cysteine and α-ketoglutaric acid in each of the forms listed in Table 1 were mixed in a molar ratio of 1:5.8 to obtain mixed powders. All of the mixed powders were prepared as white, dry powder.

### (2) Evaluation experiment

Each mixed powder and silica gel (Shin-Etsu Chemical Co., Ltd.) were stored in an incubator (CSH-122HG, Espec Co., Ltd.) at a controlled temperature of 25°C or 40°C under a light-shielded condition, and the powder was visually evaluated for solidification after 3, 7, or 14 days of the storage. In the table, the symbol "-" indicates that no solidification was observed. In the table, the symbols "+" to "+++" indicate that solidification was observed, with more symbols "+" indicating a greater degree of solidification. The specific evaluation criteria are as follows.
-: No solids (solidified lumps) are present in the mixed powder.
+: 1 to 9 Solids (solidified lumps) are present in the whole mixed powder.
++: More than 10 solids (solidified lumps) are present in the whole mixed powder, indicating that the powder is clearly solidified.
+++: The whole mixed powder is solidified, and the form as powder is not secured.

### (3) Evaluation results

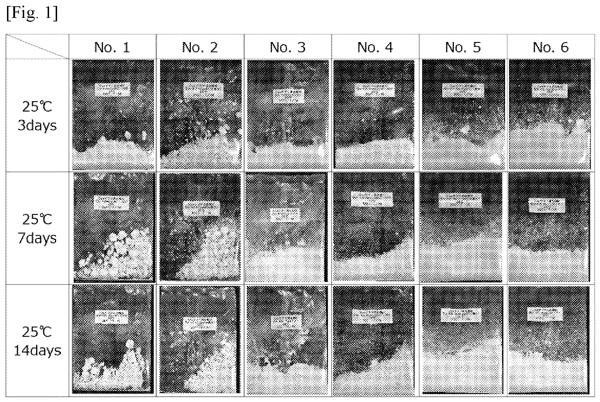

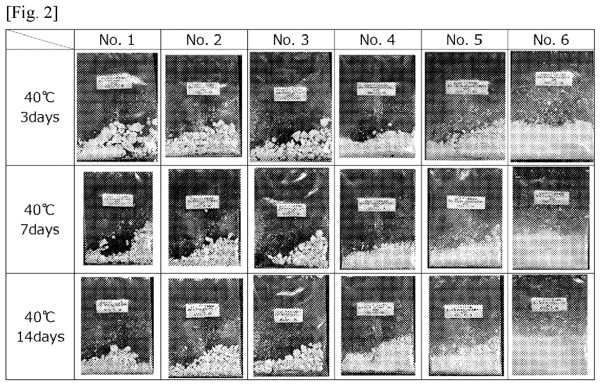

The results are shown in Table 1 and Figs. 1 and 2. In the mixed powder of L-cysteine hydrochloride monohydrate and free α-ketoglutaric acid and the mixed powder in which L-cysteine hydrochloride monohydrate was replaced with L-cysteine hydrochloride anhydrate, solidification was observed after storage at 25°C for 3 days or longer. In contrast, in the mixed powders in which L-cysteine hydrochloride monohydrate was replaced with free L-cysteine anhydrate and/or free α-ketoglutaric acid was replaced with monosodium α-ketoglutarate, no solidification was confirmed after storage at 25°C for 3 days or longer. Furthermore, in the mixed powder in which L-cysteine hydrochloride monohydrate was replaced with free L-cysteine anhydrate and free α-ketoglutaric acid was replaced with monosodium α-ketoglutarate, no solidification was observed even after storage at 40°C for 3 days or longer, after which solidification was observed in the other mixed powders.

From the above, it was revealed that the use of free L-cysteine anhydrate as L-cysteine and/or monosodium α-ketoglutarate as α-ketoglutaric acid for the mixed powder of L-cysteine and α-ketoglutaric acid improves the stability of the mixed powder (specifically, resistance to solidification). In particular, it was revealed that the use of free L-cysteine anhydrate and monosodium α-ketoglutarate markedly improves the stability (specifically, resistance to solidification) of the mixed powder.

**[Table 1]**

| Table 1: Evaluation results | | | | | | | |
|---|---|---|---|---|---|---|---|
| No. | Mixture | 25°C | | | 40°C | | |
| | | 3 days | 7 days | 14 days | 3 days | 7 days | 14 days |
| No. 1 | L-Cysteine hydrochloride monohydrate + free α-ketoglutaric acid | + | ++ | ++ | ++ | +++ | +++ |
| No. 2 | L-Cysteine hydrochloride anhydrate + free α-ketoglutaric acid | + | ++ | ++ | ++ | +++ | +++ |
| No. 3 | Free L-cysteine anhydrate + free α-ketoglutaric acid | - | - | - | ++ | ++ | +++ |
| No. 4 | L-Cysteine hydrochloride monohydrate + monosodium α-ketoglutarate | - | - | - | + | ++ | ++ |
| No. 5 | L-Cysteine hydrochloride anhydrate + monosodium α-ketoglutarate | - | - | - | + | ++ | ++ |
| No. 6 | Free L-cysteine anhydrate + monosodium α-ketoglutarate | - | - | - | - | - | - |

### Example 2: Prevention of solidification of composition

In this example, the effect of change in the form of α-ketoglutaric acid on conditions of mixed powders of various amino acid sources and α-ketoglutaric acid was evaluated.

### (1) Preparation of powders

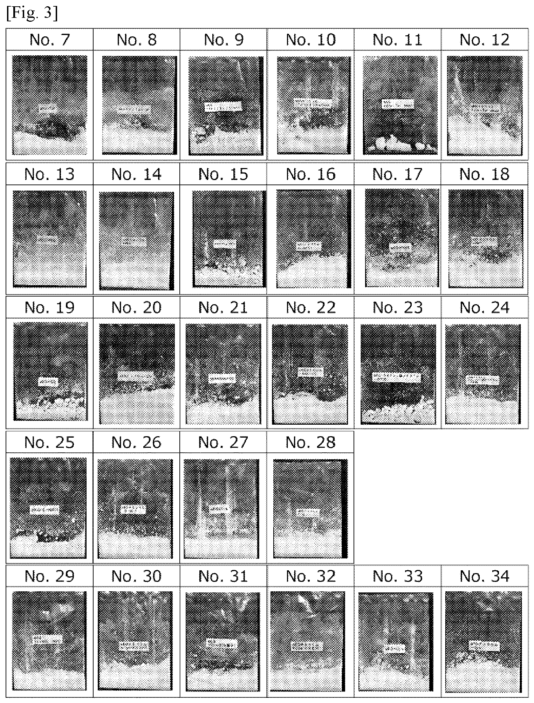

Various amino acid sources and α-ketoglutaric acid (free α-ketoglutaric acid or monosodium α-ketoglutarate) were mixed at a molar ratio of 1:1.5 to obtain mixed powders. In the mixed powder of glycyl-L-tyrosine dihydrate and free α-ketoglutaric acid and the mixed powder of L-lysine hydrochloride and free α-ketoglutaric acid, solidification was observed immediately after the preparation. All the mixed powders other than these were prepared as white, dry powder. Photographs of the mixed powders immediately after the preparation are shown in Fig. 3.

### (2) Evaluation experiment

Each powder mixture and silica gel (Shin-Etsu Chemical Co., Ltd.) were stored in an incubator (CSH-122HG, Espec Co., Ltd.) at a controlled temperature of 4°C, 25°C or 40°C under a light-shielded condition. The powders were visually evaluated for solidification after 3, 7, or 14 days of the storage. In the table, the symbol "-" indicates that no solidification was observed. In the table, the symbols "+" to "+++" indicate that solidification was observed, with more symbols "+" indicating a greater degree of solidification. The specific evaluation criteria are as follows.
-: No solids (solidified lumps) are present in the mixed powder.
+: 1 to 9 Solids (solidified lumps) are present in the whole mixed powder.
++: More than 10 solids (solidified lumps) are present in the whole mixed powder, indicating that the powder is clearly solidified.
+++: The whole mixed powder is solidified, and the form as powder is not secured.

### (3) Evaluation results

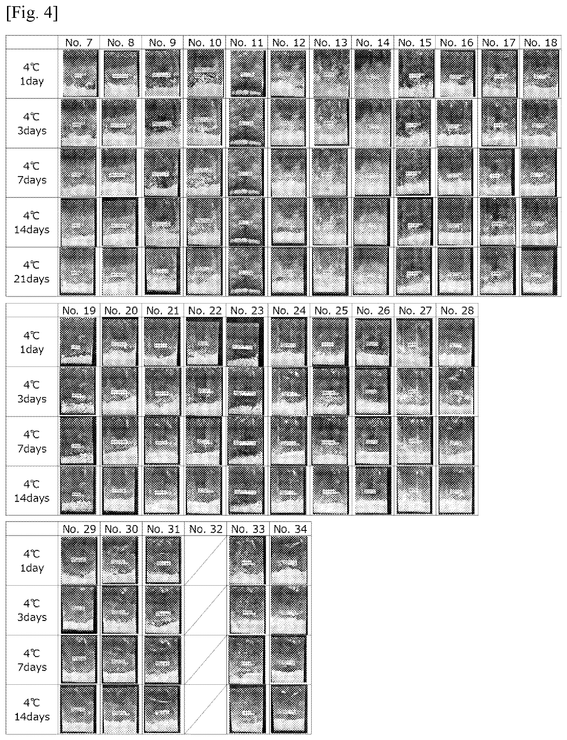

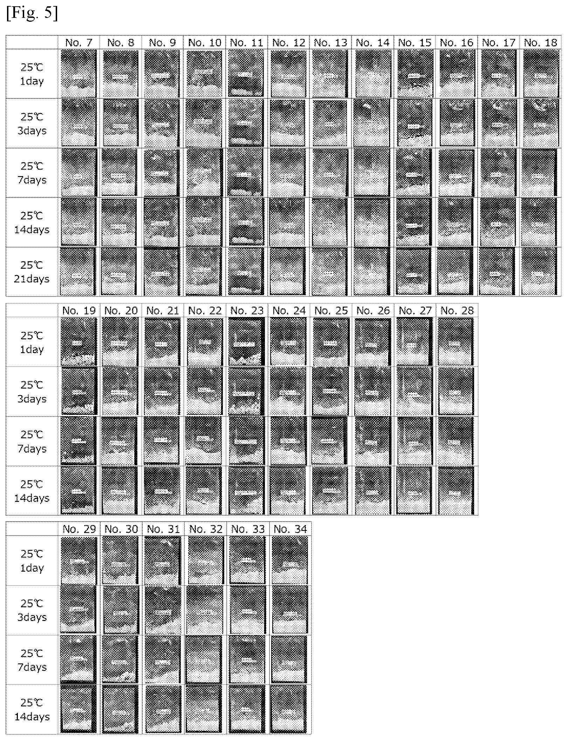

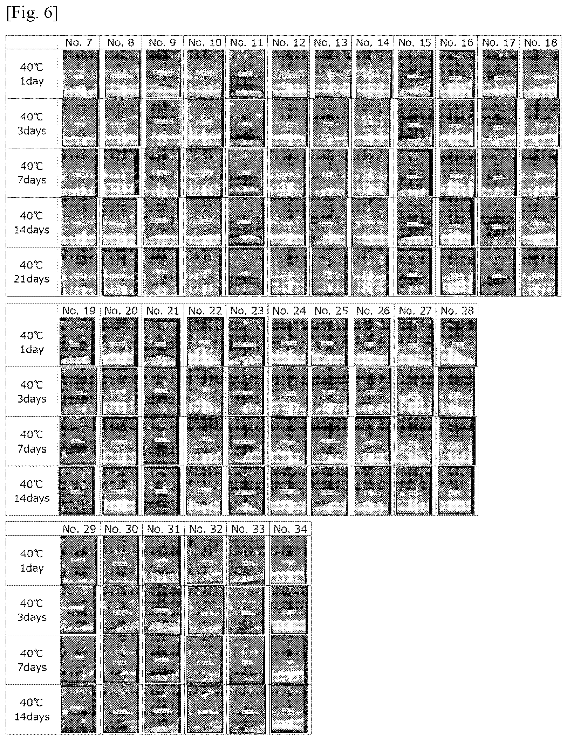

The results are shown in Tables 2 and 3 and Figs. 4 to 6. For many amino acid sources, changing free α-ketoglutaric acid to monosodium α-ketoglutarate prevented solidification of the mixed powders. The change from free α-ketoglutaric acid to monosodium α-ketoglutarate prevented solidification for, especially, the mixed powders with free glycyl-L-tyrosine dihydrate, free L-phenylalanine, L-lysine hydrochloride, free L-methionine, free glycine, free L-asparagine monohydrate, L-cysteine hydrochloride anhydrate, and free L-cysteine anhydrate.

From the above, it was revealed that the use of monosodium α-ketoglutarate as α-ketoglutaric acid improves stability (specifically, resistance to solidification) of the mixed powders of many amino acid sources and α-ketoglutaric acid, not limited to the mixed powder of L-cysteine and α-ketoglutaric acid.

**[Table 2]**

| Table 2: Evaluation results | | | | | | | | | | |
|---|---|---|---|---|---|---|---|---|---|---|
| | Mixture | 4°C | | | 25°C | | | 40°C | | |
| | | 3 days | 7 days | 14 days | 3 days | 7 days | 14 days | 3 days | 7 days | 14 days |
| No. 7 | Free L-tyrosine + free α-ketoglutaric acid | - | - | - | - | - | - | - | - | - |
| No. 8 | Free L-tyrosine + monosodium α-ketoglutarate | - | - | - | - | - | - | - | - | - |
| No. 9 | L-Tyrosine disodium salt dihydrate + free α-ketoglutaric acid | - | - | - | - | - | - | - | + | + |
| No. 10 | L-Tyrosine disodium salt dihydrate + monosodium α-ketoglutarate | - | - | - | - | - | - | - | - | - |
| No. 11 | Free glycyl-L-tyrosine dihydrate + free α-ketoglutaric acid | +++ | +++ | +++ | +++ | +++ | +++ | +++ | +++ | +++ |
| No. 12 | Free glycyl-L-tyrosine dihydrate + monosodium α-ketoglutarate | - | - | - | - | - | - | - | - | - |
| No. 13 | Free L-phenylalanine + free α-ketoglutaric acid | - | - | - | + | + | + | +++ | +++ | +++ |
| No. 14 | Free L-phenylalanine + monosodium α-ketoglutarate | - | - | - | - | - | - | - | - | - |
| No. 15 | L-Lysine hydrochloride + free α-ketoglutaric acid | ++ | ++ | ++ | ++ | +++ | +++ | +++ | +++ | +++ |
| No. 16 | L-Lysine hydrochloride + monosodium α-ketoglutarate | - | - | - | - | - | - | - | - | - |
| No. 17 | Free L-methionine + free α-ketoglutaric acid | + | + | + | + | + | + | ++ | +++ | +++ |
| No. 18 | Free L-methionine + monosodium α-ketoglutarate | - | - | - | - | - | - | - | - | - |
| No. 19 | Free glycine + free α-ketoglutaric acid | +++ | +++ | +++ | +++ | +++ | +++ | +++ | +++ | +++ |
| No. 20 | Free glycine + monosodium α-ketoglutarate | - | - | - | - | - | - | - | - | - |

**[Table 3]**

| Table 3: Evaluation results | | | | | | | | | | |
|---|---|---|---|---|---|---|---|---|---|---|
| | Mixture | 4°C | | | 25°C | | | 40°C | | |
| | | 3 days | 7 days | 14 days | 3 days | 7 days | 14 days | 3 days | 7 days | 14 days |
| No. 21 | Free L-asparagine + free α-ketoglutaric acid | - | - | - | + | ++ | ++ | +++ | +++ | +++ |
| No. 22 | Free L-asparagine monohydrate + monosodium α-ketoglutarate | - | - | - | - | - | - | - | - | - |
| No. 23 | Monosodium L-glutamate monohydrate + free α-ketoglutaric acid | +++ | +++ | +++ | +++ | +++ | +++ | ++ | +++ | +++ |
| No. 24 | Monosodium L-glutamate monohydrate + monosodium α-ketoglutarate | - | - | - | - | - | - | - | - | - |
| No. 25 | L-Omithine hydrochloride + free α-ketoglutaric acid | - | - | - | + | + | + | + | + | + |
| No. 26 | L-Omithine hydrochloride + monosodium α-ketoglutarate | - | - | - | - | - | - | - | - | - |
| No. 27 | Free L-tryptophan + free α-ketoglutaric acid | - | - | - | - | - | - | - | - | - |
| No. 28 | Free L-tryptophan + monosodium α-ketoglutarate | - | - | - | - | - | - | - | - | - |
| No. 29 | L-Cysteine hydrochloride monohydrate + free α-ketoglutaric acid | - | - | - | ++ | ++ | +++ | +++ | +++ | +++ |
| No. 30 | L-Cysteine hydrochloride monohydrate + monosodium α-ketoglutarate | - | - | - | +++ | +++ | +++ | +++ | +++ | +++ |
| No. 31 | L-Cysteine hydrochloride anhydrate + free α-ketoglutaric acid | - | - | - | ++ | ++ | ++ | ++ | +++ | +++ |
| No. 32 | L-Cysteine hydrochloride anhydrate + monosodium α-ketoglutarate | | | | - | - | - | +++ | +++ | +++ |
| No. 33 | Free L-cysteine anhydrate + free α-ketoglutaric acid | - | - | - | - | - | - | +++ | +++ | +++ |
| No. 34 | Free L-cysteine anhydrate + monosodium α-ketoglutarate | - | - | - | - | - | - | - | - | - |

### Example 3: Prevention of decrease in L-Cysteine content in compositions

In this experimental example, the effect of change in the forms of L-cysteine and/or α-ketoglutaric acid in mixed powder of L-cysteine and α-ketoglutaric acid on the transition of L-cysteine content in the powder was evaluated.

### (1) Preparation of powders

L-Cysteine (L-cysteine hydrochloride monohydrate or free L-cysteine anhydrate) and α-ketoglutaric acid (free α-ketoglutaric acid or monosodium α-ketoglutarate) were mixed at a molar ratio of 1:4.9 to obtain mixed powders. All of the mixed powders were prepared as white, dry powder.

### (2) Evaluation experiment

Each mixed powder and silica gel (Shin-Etsu Chemical Co., Ltd.) were stored in an incubator (CSH-122HG, Espec Co., Ltd.) at a controlled temperature of 25°C or 40°C under a light-shielded condition, and the L-cysteine content in the mixed powder was analyzed over time according to the method described in WO2021/060517A.

### (3) Evaluation results

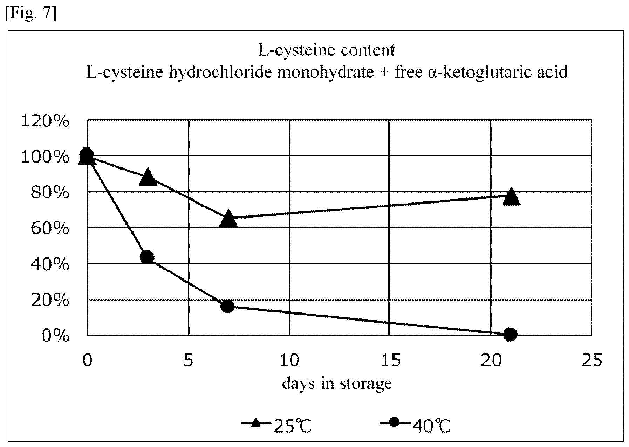

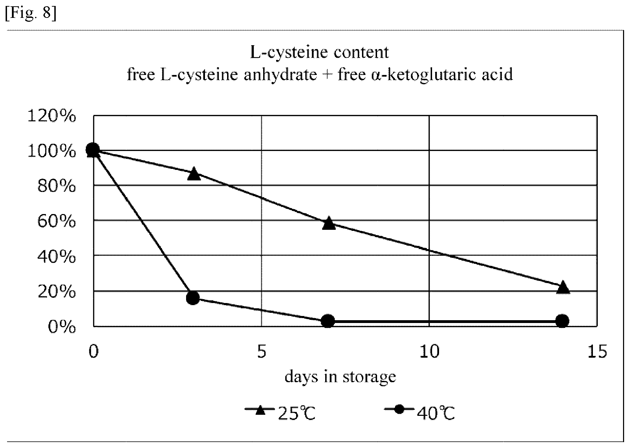

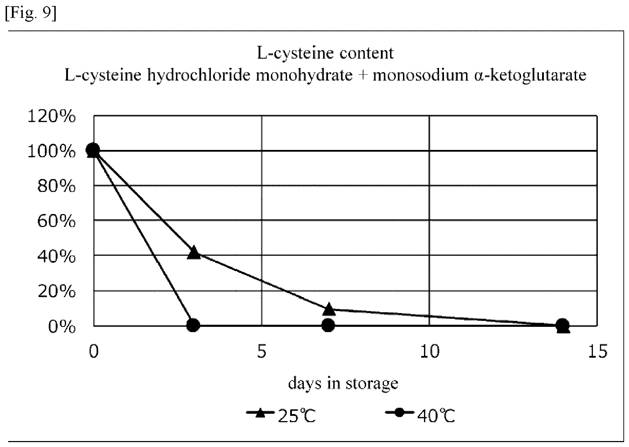

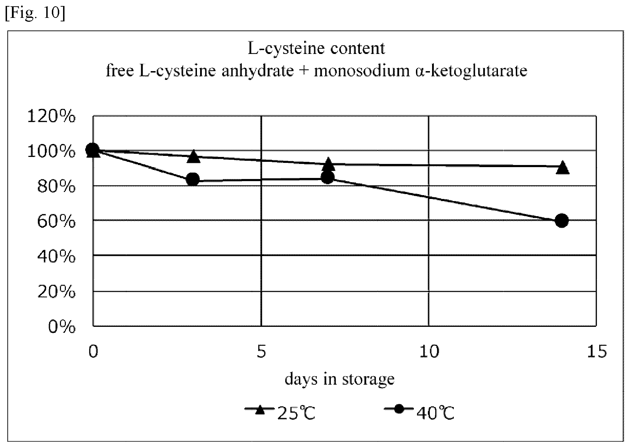

The results are shown in Figs. 7 to 10. In the mixed powders containing free α-ketoglutaric acid, decrease in L-cysteine content was observed after storage at 25°C or 40°C, regardless of whether the L-cysteine was L-cysteine hydrochloride monohydrate or free L-cysteine anhydrate, and L-cysteine was no longer detectable after storage at 40°C for 7 days or longer. On the other hand, decrease in L-cysteine content was greatly improved in the mixed powder containing free L-cysteine and monosodium α-ketoglutarate.

From the above, it was revealed that the use of free L-cysteine anhydrate as L-cysteine and monosodium α-ketoglutarate as α-ketoglutaric acid in a mixed powder of L-cysteine and α-ketoglutaric acid improves stability of the mixed powder (specifically, stability of L-cysteine).

### Industrial applicability

According to the present invention, stability of a composition containing an amino acid source and α-ketoglutaric acid can be improved. According to the present invention, animal cells can be cultured by using the composition.

## Claims

1. A composition for animal cell culture, which
comprises an amino acid source and α-ketoglutaric acid, and
wherein the composition satisfies the following conditions (A) and/or (B);
(A) the α-ketoglutaric acid is a salt of α-ketoglutaric acid; and/or
(B) the amino acid source is free L-cysteine anhydrate.

2. The composition according to claim 1, wherein the composition satisfies at least the condition (A).

3. The composition according to claim 1 or 2, wherein the composition is a powder form.

4. The composition according to any one of claims 1 to 3, wherein the composition is a culture medium or culture medium additive.

5. The composition according to claim 4, wherein the culture medium is a basal medium, a feed medium, or a perfusion medium.

6. The composition according to any one of claims 1 to 5, wherein the salt of α-ketoglutaric acid is an alkali metal salt or alkaline earth metal salt of α-ketoglutaric acid.

7. The composition according to any one of claims 1 to 6, wherein the salt of α-ketoglutaric acid is a sodium salt or potassium salt of α-ketoglutaric acid.

8. The composition according to any one of claims 1 to 7, wherein the salt of α-ketoglutaric acid is monosodium α-ketoglutarate or disodium α-ketoglutarate.

9. The composition according to any one of claims 1 to 8, wherein the amino acid source is cysteine, glycine, asparagine, glutamic acid, lysine, phenylalanine, methionine, ornithine, tyrosine, or a glycine-containing dipeptide.

10. The composition according to claim 9, wherein the composition satisfies one or more conditions selected from the group consisting of the following conditions (1) to (10):
(1) the cysteine is free L-cysteine or L-cysteine hydrochloride;
(2) the glycine is free glycine or free glycyl-L-tyrosine;
(3) the asparagine is free L-asparagine;
(4) the glutamic acid is monosodium L-glutamate;
(5) the lysine is L-lysine hydrochloride;
(6) the phenylalanine is free L-phenylalanine;
(7) the methionine is free L-methionine;
(8) the ornithine is L-ornithine hydrochloride;
(9) the tyrosine is L-tyrosine disodium salt; and
(10) the glycine-containing dipeptide is free glycyl-L-tyrosine.

11. The composition according to claim 9 or 10, wherein the composition satisfies one or more conditions selected from the group consisting of the following conditions (1a) to (10a):
(1a) the cysteine is free L-cysteine anhydrate or L-cysteine hydrochloride monohydrate;
(2a) the glycine is free glycine or free glycyl-L-tyrosine;
(3a) the asparagine is free L-asparagine monohydrate;
(4a) the glutamic acid is monosodium L-glutamate monohydrate;
(5a) the lysine is L-lysine hydrochloride;
(6a) the phenylalanine is free L-phenylalanine;
(7a) the methionine is free L-methionine;
(8a) the ornithine is L-ornithine hydrochloride;
(9a) the tyrosine is L-tyrosine disodium salt dihydrate; and
(10a) the glycine-containing dipeptide is free glycyl-L-tyrosine dihydrate.

12. The composition according to any one of claims 1 to 11, wherein the amino acid source is cysteine.

13. The composition according to any one of claims 9 to 12, wherein the cysteine is free L-cysteine anhydrate or L-cysteine hydrochloride monohydrate.

14. The composition according to any one of claims 9 to 13, wherein the cysteine is free L-cysteine anhydrate.

15. The composition according to any one of claims 1 to 14, wherein content of the α-ketoglutaric acid is 1 to 20 times content of the amino acid source in terms of molar ratio.

16. A method for producing a target substance, which comprises
culturing animal cells having an ability to produce the target substance using the composition according to any one of claims 1 to 15; and
collecting the target substance.

17. The method according to claim 16, wherein the target substance is a protein or virus.

18. A method for culturing animal cells, which comprises culturing animal cells by using the composition according to any one of claims 1 to 15.

19. Use of a salt of α-ketoglutaric acid for preventing solidification of a composition containing an amino acid source.

20. The use according to claim 19, wherein the amino acid source is cysteine, glycine, asparagine, glutamic acid, lysine, phenylalanine, methionine, ornithine, tyrosine, or a glycine-containing dipeptide.

21. The use according to claim 20, wherein one or more conditions selected from the following conditions (1) to (10) is/are satisfied:
(1) the cysteine is free L-cysteine or L-cysteine hydrochloride;
(2) the glycine is free glycine or free glycyl-L-tyrosine;
(3) the asparagine is free L-asparagine;
(4) the glutamic acid is monosodium L-glutamate;
(5) the lysine is L-lysine hydrochloride;
(6) the phenylalanine is free L-phenylalanine;
(7) the methionine is free L-methionine;
(8) the ornithine is L-ornithine hydrochloride;
(9) the tyrosine is L-tyrosine disodium salt; and
(10) the glycine-containing dipeptide is free glycyl-L-tyrosine.

22. The use according to claim 20 or 21, wherein one or more conditions selected from the following conditions (1a) to (10a) is/are satisfied:
(1a) the cysteine is free L-cysteine anhydrate or L-cysteine hydrochloride monohydrate;
(2a) the glycine is free glycine or free glycyl-L-tyrosine;
(3a) the asparagine is free L-asparagine monohydrate;
(4a) the glutamic acid is monosodium L-glutamate monohydrate;
(5a) the lysine is L-lysine hydrochloride;
(6a) the phenylalanine is free L-phenylalanine;
(7a) the methionine is free L-methionine;
(8a) the ornithine is L-ornithine hydrochloride;
(9a) the tyrosine is L-tyrosine disodium salt dihydrate; and
(10a) the glycine-containing dipeptide is free glycyl-L-tyrosine dihydrate.

23. The use according to any one of claims 19 to 22, wherein the amino acid source is cysteine.

24. Use of a salt of α-ketoglutaric acid for preventing decrease in cysteine content in a composition containing cysteine.

25. The use according to any one of claims 19 to 24, wherein the salt of α-ketoglutaric acid is an alkali metal salt or alkaline earth metal salt of α-ketoglutaric acid.

26. The use according to any one of claims 19 to 25, wherein the salt of α-ketoglutaric acid is a sodium salt or potassium salt of α-ketoglutaric acid.

27. The use according to any one of claims 19 to 26, wherein the salt of α-ketoglutaric acid is monosodium α-ketoglutarate or disodium α-ketoglutarate.

28. The use according to any one of claims 20 to 27, wherein the cysteine is free L-cysteine anhydrate or L-cysteine hydrochloride monohydrate.

29. The use according to any one of claims 20 to 28, wherein the cysteine is free L-cysteine anhydrate.

30. The use according to any one of claims 19 to 29, wherein the salt of α-ketoglutaric acid is used so that content of the salt of α-ketoglutaric acid in the composition is 1 to 20 times content of the amino acid source or the cysteine in the composition in terms of molar ratio.

31. A method for producing a composition for animal cell culture, wherein
the composition is the composition according to any one of claims 1 to 15, and
the method comprises mixing the amino acid source and the α-ketoglutaric acid.
